(19) **Europäisches Patentamt**
**European Patent Office**
**Office européen des brevets**

(11) **EP 4 525 724 B1**

(12) **EUROPEAN PATENT SPECIFICATION**

(45) Date of publication and mention
of the grant of the patent:
**20.05.2026  Bulletin 2026/21**

(21) Application number: **22730394.8**

(22) Date of filing: **18.05.2022**

(51) International Patent Classification (IPC):
***A61B 6/00*** *(2024.01)*      ***A61B 6/40*** *(2024.01)*
***A61B 6/42*** *(2024.01)*

(52) Cooperative Patent Classification (CPC):
**A61B 6/4429; A61B 6/4014; A61B 6/405;
A61B 6/4241; A61B 6/4266; A61B 6/482;
A61B 6/488; A61B 6/544**

(86) International application number:
**PCT/EP2022/063403**

(87) International publication number:
**WO 2023/222211 (23.11.2023 Gazette 2023/47)**

(54) **RADIOLOGICAL IMAGING METHOD WITH A MULTI-ENERGY SCOUT VIEW**

RADIOLOGISCHES BILDGEBUNGSVERFAHREN MIT MEHRFACHENERGIE-SCOUT-ANSICHT

MÉTHODE D'IMAGERIE RADIOLOGIQUE AVEC IMAGE DE SCANNOGRAMME À PLUSIEURS
NIVEAUX D'ÉNERGIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.03.2025  Bulletin 2025/13**

(73) Proprietor: **EOS Imaging
75009 Paris (FR)**

(72) Inventors:
• **BEUCHER, Jérôme
77450 ESBLY (FR)**

• **DÉSAUTÉ, Pascal
75020 PARIS (FR)**
• **MORICHAU-BEAUCHANT, Pierre
75012 PARIS (FR)**
• **RESHEF, Aymeric
75014 PARIS (FR)**

(74) Representative: **Plasseraud IP
104 Rue de Richelieu
CS92104
75080 Paris Cedex 02 (FR)**

(56) References cited:
**WO-A1-2021/094004      US-A1- 2019 246 999**

**Description**

FIELD OF THE INVENTION

The invention relates to the technical field of radiological imaging method and of radiological apparatus for performing this radiological method.

BACKGROUND OF THE INVENTION

Different types of radiological images can be done, among which:

- radiological scan image dedicated to a total (detailed and complete) view, of a patient or of a patient organ or of a part of a patient organ, which is used for diagnosis by practitioner,
- radiological scan image dedicated to bone density distribution within a patient which is used for bone density evaluation by practitioner, of a patient or of a patient organ or of a part of a patient organ.

Radiological image is preferably X-ray image.

In order to improve accuracy of diagnosis and/or bone density evaluation, in a first step a scout view is performed, and then using information extracted from this scout view to adapt imaging parameters, the scan image is performed which is then used by the practitioner, either for diagnosis or for bone density evaluation.

Scout view and scan image are performed by vertical scanning along the height of a standing patient, of a frontal image taking-line including a frontal radiation source and a frontal radiation detector and/or of a lateral image taking-line including a lateral radiation source and a lateral radiation detector.

According to a first prior art, when performing a mono-energy scout view followed by a mono-energy scan image, imaging parameters are adapted so as to allow to get at a good quality diagnosis image but which could not give good result for bone density evaluation, i.e. from which diagnosis image no good result for bone density evaluation can be derived. Then, if bone density evaluation is also needed, not only a new scan image with different imaging parameters should be done, but also this new scan image cannot topologically correspond exactly to the former scan image, because the standing patient would have moved, at least a little, in between.

According to a second prior art, performing a multi-energy scan image, imaging parameters are adapted so as to allow to get a good quality bone density image but which could not give good result for diagnosis, i.e. from which bone density image no good result for diagnosis image can be derived. Then, if diagnosis is also needed, not only a new scan image with different imaging parameters should be done, but also this new scan image cannot topologically correspond exactly to the former scan image, because the standing patient would have moved, at least a little, in between.

WO2021094004A1 may be considered to disclose a radiological imaging method comprising: 2 radiation sources with imaging directions orthogonal to each other, one frontal radiation source and one lateral radiation source, sliding vertically so as to perform vertical scanning of a standing patient along a vertical scanning direction, wherein said radiological method comprises at least one operating mode in which: a frontal scout view is made by performing a preliminary vertical scanning of a standing patient along said vertical scanning direction by said frontal radiation source, said frontal scout view is processed to identify a specific bone localization within said frontal scout view, a driving current intensity of at least said frontal radiation source is modulated along said vertical scanning direction, depending on patient thickness and on said identified specific bone localization along said vertical scanning direction, with no modulation of driving voltage intensity of said frontal radiation source along said vertical scanning direction, said driving current intensity modulation of said frontal radiation source is performed automatically, so as to improve a compromise between: lowering the global radiation dose received by a patient during said vertical scanning, while keeping at a sufficient level the local image contrasts of said identified specific bone localization at different imaging positions along said vertical scanning direction, for the frontal image; and a radiological imaging method comprising: 2 radiation sources with imaging directions orthogonal to each other, one frontal radiation source and one lateral radiation source, sliding vertically so as to perform vertical scanning of a standing patient along a vertical scanning direction, wherein said radiological method comprises at least one operating mode in which: a lateral scout view is made by performing a preliminary vertical scanning of a standing patient along said vertical scanning direction by said lateral radiation source, said lateral scout view is processed to identify a specific bone localization within said lateral scout view, a driving current intensity of at least said lateral radiation source is modulated along said vertical scanning direction, depending on patient thickness and on said identified specific bone localization along said vertical scanning direction, with no modulation of driving voltage intensity of said lateral radiation source along said vertical scanning direction, said driving current intensity modulation of said lateral radiation source is performed automatically, so as to improve a compromise between: lowering the global radiation dose received by a patient during said vertical scanning, while keeping at a sufficient level the local image contrasts of said identified specific bone localization at different imaging positions along said vertical scanning direction, for the lateral image; and a radiological imaging method comprising: 2 radiation sources with imaging directions orthogonal to each other, one frontal radiation source and one lateral radiation source, sliding vertically so as to perform vertical scanning of a standing patient along a vertical scanning direction, wherein said radiological method comprises at least one operating mode in which: frontal and lateral scout views are made by performing a preliminary vertical scanning of a standing patient along said vertical scanning direction by said frontal and lateral radiation sources, said frontal and lateral scout views are processed to identify a specific bone localization within both said frontal and lateral scout views, driving current intensities of both said frontal and lateral radiation sources are modulated along said vertical scanning direction, depending on patient thickness and on said identified specific bone localization along said vertical scanning direction, with no modulation of driving voltage intensities of either frontal or lateral radiation sources along said vertical scanning direction, said driving current intensity modulation of said frontal radiation source, as well as said driving current intensity modulation of said lateral radiation source, are all performed simultaneously, preferably synchronously, and automatically, so as to improve a compromise between: lowering the global radiation dose received by a patient during said vertical scanning, while keeping at a sufficient level the local image contrasts of said identified specific bone localization at different imaging positions along said vertical scanning direction, for the frontal image and for the lateral image.

SUMMARY OF THE INVENTION

The object of the present invention is to alleviate at least partly the above mentioned drawbacks.

More particularly, the invention aims at providing for a scan image which can be used for diagnosis with good result, but also:

- either, partial scout views can be extracted and further combined from which multi-energy scout view, so as to give correct result for bone density evaluation too, at the cost of a simple multi-energy view and at the cost of a lower radiation dose too (scout view is performed with about 10 times less radiation dose or even less, as compared to scan image). A combination of both partial scout views will lead to accurate evaluation of bone density. So, good diagnosis and correct bone density evaluation can be then both derived from the scan image and from its scout view in a rather simple way in a single global operation.
- or from which, at the same time, partial images can be extracted and further combined so as to give good result for bone density evaluation too. A combination of both partial images will lead to accurate evaluation of bone density. So, good diagnosis and good bone density evaluation can be then both derived from the same scan image. Besides, since diagnosis and good bone density evaluation are both derived from the same scan image, there will be an exact topological correspondence between diagnosis and good bone density evaluation, because the standing patient is exactly in the same position for both.

Therefore, in order to be useful, with good quality, both for diagnosis and for bone density evaluation, the radiological method uses:

- first, a scout view which is multi-energy, this scout view being only mono-energy in prior art,
- second, extracted information from this multi-energy scout view, which information is used to then perform an improved scan image, whether mono-energy or multi-energy scan image, this scan image being only mono-energy in prior art.

The multi-energy scout view will give even better results if performed before a multi-energy scan image rather than before a mono-energy scan image.

The multi-energy scout view can be either a frontal scout view, or a lateral scout view, or include both frontal and lateral scout views.

A first object of the invention deals with a frontal mono-energy or multi-energy scan image, performed after a frontal multi-energy scout view.

This first object is achieved with a radiological imaging method comprising:

➢ 2 radiation sources with imaging directions orthogonal to each other, one frontal radiation source and one lateral radiation source, sliding vertically so as to perform vertical scanning of a standing patient along a vertical scanning direction,

➢ 2 radiation detectors which are respectively associated with said 2 radiations sources, one frontal radiation detector and one lateral radiation detector, sliding vertically so as to perform vertical scanning of a standing patient along said vertical scanning direction, at least said frontal radiation detector being a multi-energy counting detector,

wherein said radiological method comprises at least one operating mode in which:

➢ a frontal multi-energy scout view is made by performing a preliminary vertical scanning of a standing patient along said vertical scanning direction by said frontal radiation source and by said frontal radiation detector, so that said frontal radiation detector gives at least:

◦ a first frontal scout view corresponding to a first portion of energy which is received by said frontal radiation detector and which is below a first given energy threshold, called low energy frontal scout view,
◦ a second frontal scout view corresponding to a second portion of energy which is received by said frontal radiation detector and which is above a second given energy threshold, called high energy frontal scout view,

➢ said first frontal scout view and said second frontal scout view are combined and processed so as to evaluate:

◦ at least a patient bone thickness,
◦ at least a patient soft tissue thickness,
◦ a patient specific bone localization at different imaging positions along said vertical scanning direction,

➢ a frontal image is made by performing a vertical scanning of a standing patient along said vertical scanning direction by said frontal radiation source and by said frontal radiation detector, with:

◦ a modulation of a driving current intensity of at least said frontal radiation source along said vertical scanning direction, depending on said patient bone thickness, on said patient soft tissue thickness, and on said patient specific bone localization at different imaging positions along said vertical scanning direction,
◦ and preferably also a modulation of a driving voltage intensity of said frontal radiation source along said vertical scanning direction, depending on said patient bone thickness, on said patient soft tissue thickness, and on said patient specific bone localization at different imaging positions along said vertical scanning direction,
◦ either driving current intensity modulation of said frontal radiation source, with no voltage intensity modulation of said frontal radiation source, is performed automatically, so as to improve a compromise between:

▪ the global radiation dose received by a patient during said vertical scanning,
▪ and the local image contrasts of said identified specific bone(s) localization at different imaging positions along said vertical scanning direction, for the frontal image,

◦ or both driving current intensity and voltage intensity modulations of said frontal radiation source are performed simultaneously, preferably synchronously, and automatically, so as to improve a compromise between:

▪ the global radiation dose received by a patient during said vertical scanning,
▪ and the local image contrasts of said identified specific bone(s) localization at different imaging positions along said vertical scanning direction, for the frontal image.

A second object of the invention deals with a lateral mono-energy or multi-energy scan image, performed after a lateral multi-energy scout view.

This second object is achieved with a radiological imaging method comprising:

➢ 2 radiation sources with imaging directions orthogonal to each other, one frontal radiation source and one lateral radiation source, sliding vertically so as to perform vertical scanning of a standing patient along a vertical scanning direction,
➢ 2 radiation detectors which are respectively associated with said 2 radiations sources, one frontal radiation detector and one lateral radiation detector, sliding vertically so as to perform vertical scanning of a standing patient along said vertical scanning direction, at least said lateral radiation detector being a multi-energy counting detector,

wherein said radiological method comprises at least one operating mode in which:

➢ a lateral multi-energy scout view is made by performing a preliminary vertical scanning of a standing patient along said vertical scanning direction by said lateral radiation source and by said lateral radiation detector, so that said lateral radiation detector gives at least:

◦ a first lateral scout view corresponding to a first portion of energy which is received by said lateral radiation detector and which is below a first given energy threshold, called low energy lateral scout view,
◦ a second lateral scout view corresponding to a second portion of energy which is received by said lateral radiation detector and which is above a second given energy threshold, called high energy lateral scout view,

➢ said first lateral scout view and said second lateral scout view are combined and processed so as to evaluate:

○ at least a patient bone thickness,
○ at least a patient soft tissue thickness,
○ a patient specific bone localization at different imaging positions along said vertical scanning direction,

➢ a lateral image is made by performing a vertical scanning of a standing patient along said vertical scanning direction by said lateral radiation source and by said lateral radiation detector, with:

○ a modulation of a driving current intensity of at least said lateral radiation source along said vertical scanning direction, depending on said patient bone thickness, on said patient soft tissue thickness, and on said patient specific bone localization at different imaging positions along said vertical scanning direction,
○ and preferably also a modulation of a driving voltage intensity of said lateral radiation source along said vertical scanning direction, depending on said patient bone thickness, on said patient soft tissue thickness, and on said patient specific bone localization at different imaging positions along said vertical scanning direction,
○ either driving current intensity modulation of said lateral radiation source, with no voltage intensity modulation of said lateral radiation source, is performed automatically, so as to improve a compromise between:

■ the global radiation dose received by a patient during said vertical scanning,
■ and the local image contrasts of said identified specific bone(s) localization at different imaging positions along said vertical scanning direction, for the lateral image,

○ or both driving current intensity and voltage intensity modulations of said lateral radiation source are performed simultaneously, preferably synchronously, and automatically, so as to improve a compromise between:

■ the global radiation dose received by a patient during said vertical scanning,
■ and the local image contrasts of said identified specific bone(s) localization at different imaging positions along said vertical scanning direction, for the lateral image.

A third object of the invention deals with both frontal and lateral, either mono-energy or multi-energy, scan images, performed after both frontal and lateral multi-energy scout views.
This third object is achieved with a radiological imaging method comprising:

➢ 2 radiation sources with imaging directions orthogonal to each other, one frontal radiation source and one lateral radiation source, sliding vertically so as to perform vertical scanning of a standing patient along a vertical scanning direction,
➢ 2 radiation detectors which are respectively associated with said 2 radiations sources, one frontal radiation detector and one lateral radiation detector, sliding vertically so as to perform vertical scanning of a standing patient along said vertical scanning direction, said 2 radiation detectors being respectively 2 multi-energy counting detectors,

wherein said radiological method comprises at least one operating mode in which:

➢ frontal and lateral multi-energy scout views are made by performing a preliminary vertical scanning of a standing patient along said vertical scanning direction by said frontal and lateral radiation sources and by said frontal and lateral radiation detectors, so that said frontal and lateral radiation detectors give at least:

○ a first frontal scout view corresponding to a first portion of energy which is received by said frontal radiation detector and which is below a first given energy threshold, called low energy frontal scout view,
○ a second frontal scout view corresponding to a second portion of energy which is received by said frontal radiation detector and which is above a second given energy threshold, called high energy frontal scout view,
○ a first lateral scout view corresponding to a first portion of energy which is received by said lateral radiation detector and which is below a first given energy threshold, called low energy lateral scout view,
○ a second lateral scout view corresponding to a second portion of energy which is received by said lateral radiation detector and which is above a second given energy threshold, called high energy lateral scout view,

➢ said first frontal and lateral scout views and said second frontal and lateral scout views are combined and processed so as to evaluate:

  ◦ at least a patient bone thickness,
  ◦ at least a patient soft tissue thickness,
  ◦ a patient specific bone localization at different imaging positions along said vertical scanning direction,

➢ a frontal image is made by performing a vertical scanning of a standing patient along said vertical scanning direction by said frontal radiation source and by said frontal radiation detector, and a lateral image is made by performing a vertical scanning of a standing patient along said vertical scanning direction by said lateral radiation source and by said lateral radiation detector, both frontal and lateral images being made during same vertical scanning, with:

  ◦ a modulation of driving current intensities of both said frontal and lateral radiation sources along said vertical scanning direction, depending on said patient bone thickness, on said patient soft tissue thickness, and on said patient specific bone localization at different imaging positions along said vertical scanning direction,
  ◦ and preferably also a modulation of driving voltage intensities of both said frontal and lateral radiation sources along said vertical scanning direction, depending on said patient bone thickness, on said patient soft tissue thickness, and on said patient specific bone localization at different imaging positions along said vertical scanning direction,
  ◦ either driving current intensity modulation of said frontal radiation source, with no voltage intensity modulation of said frontal radiation source, as well as driving current intensity modulation of said lateral radiation source, with no voltage intensity modulation of said lateral radiation source, are performed simultaneously, preferably synchronously, and automatically, so as to improve a compromise between:

    ▪ the global radiation dose received by a patient during said vertical scanning,
    ▪ and the local image contrasts of said identified specific bone(s) localization at different imaging positions along said vertical scanning direction, for the frontal image and for the lateral image,

  ◦ or both driving current intensity and voltage intensity modulations of said frontal radiation source, are performed simultaneously, preferably synchronously, and automatically, as well as both driving current intensity and voltage intensity modulations of said lateral radiation source, are performed simultaneously, preferably synchronously, and automatically, so as to improve a compromise between:

    ▪ the global radiation dose received by a patient during said vertical scanning,
    ▪ and the local image contrasts of said identified specific bone(s) localization at different imaging positions along said vertical scanning direction, for the frontal image and for the lateral image.

Preferred embodiments comprise one or more of the following features, which can be taken separately or together, either in partial combination or in full combination, with any of preceding objects of the invention.

Preferably,

➢ said frontal multi-energy scout view is made by performing a single preliminary vertical scanning of a standing patient along said vertical scanning direction by said frontal radiation source and by said frontal radiation detector, so that said frontal radiation detector gives at least:

  ◦ said first frontal scout view,
  ◦ said second frontal scout view.

➢ said lateral multi-energy scout view is made by performing a single preliminary vertical scanning of a standing patient along said vertical scanning direction by said lateral radiation source and by said lateral radiation detector, so that said lateral radiation detector gives at least:

  ◦ said first lateral scout view,
  ◦ said second lateral scout view.

➢ both said frontal multi-energy scout view and said lateral multi-energy scout view being made during same single preliminary vertical scanning.

Hence, since frontal and lateral scout views are both performed simultaneously during same single vertical scanning, frontal and lateral scout views will topologically correspond exactly to each other, because the standing patient would not have moved in between.

Preferably,

➢ said frontal image is made by performing a single vertical scanning of a standing patient along said vertical scanning direction by said frontal radiation source and by said frontal radiation detector,
➢ said lateral image is made by performing a single vertical scanning of a standing patient along said vertical scanning direction by said lateral radiation source and by said lateral radiation detector,
➢ both said frontal image and said lateral image being made during same single vertical scanning.

Hence, since frontal and lateral images are both performed simultaneously during same vertical scanning, frontal and lateral images will topologically correspond exactly to each other, because the standing patient would not have moved in between.

Preferably, said first given energy threshold is equal to or less than said second given energy threshold, preferably equal to said second given energy threshold.

Hence, in both cases all the range of energy threshold is covered, and in the second case at lower cost.

Preferably,

➢ said first given energy threshold is equal to said second given energy threshold,
➢ said frontal and/or lateral multi-energy scout views are made so that said frontal and/or lateral radiation detectors first give:

◦ said first frontal scout view,
◦ a third frontal scout view corresponding to the whole energy which is received by said frontal radiation detector, called total energy frontal scout view,

■ said second frontal scout view being obtained by a subtracting said first frontal scout view from said third frontal scout view,

◦ and/or said first lateral scout view,
◦ and/or a third lateral scout view corresponding to the whole energy which is received by said lateral radiation detector, called total energy lateral scout view,

■ said second lateral scout view being obtained by a subtracting said first lateral scout view from said third lateral scout view.

Hence, total energy scout view and high energy scout view can be given directly by the detector, whereas low energy scout view can be obtained by a simple subtraction, by subtracting high energy scout view from total energy scout view.

Preferably,

➢ said first frontal and/or lateral scout view(s) and said second frontal and/or lateral scout view(s) are combined and processed so as to evaluate a patient bone thickness profile along said vertical scanning direction,
➢ and/or said first frontal and/or lateral scout view(s) and said second frontal and/or lateral scout view(s) are combined and processed so as to evaluate a patient soft tissue thickness profile along said vertical scanning direction,
➢ said frontal image is made by performing a vertical scanning of a standing patient along said vertical scanning direction by said frontal radiation source and by said frontal radiation detector, with:

◦ a modulation of a driving current intensity of at least said frontal radiation source along said vertical scanning direction, depending on said patient bone thickness profile and/or on said patient soft tissue thickness profile along said vertical scanning direction,
◦ and preferably also said modulation of a driving voltage intensity of said frontal radiation source along said vertical scanning direction, depending on said patient bone thickness profile and on said patient soft tissue thickness profile along said vertical scanning direction,

➢ said lateral image is made by performing a vertical scanning of a standing patient along said vertical scanning direction by said lateral radiation source and by said lateral radiation detector, with:

○ a modulation of a driving current intensity of at least said lateral radiation source along said vertical scanning direction, depending on said patient bone thickness profile and/or on said patient soft tissue thickness profile along said vertical scanning direction,

○ and preferably also said modulation of a driving voltage intensity of said lateral radiation source along said vertical scanning direction, depending on said patient bone thickness profile and on said patient soft tissue thickness profile along said vertical scanning direction,

➢ both said frontal image and said lateral image being made during same vertical scanning.

Hence, since frontal and lateral images are both performed simultaneously during same vertical scanning, frontal and lateral images will topologically correspond exactly to each other, because the standing patient would have not moved in between.

Preferably,

○ either said driving current intensity modulation(s) of said frontal and/or lateral radiation source(s), with no voltage intensity modulation of said frontal and/or lateral radiation source(s), is performed automatically, so as to improve a compromise between:

   ▪ lowering the global radiation dose received by a patient during said vertical scanning,
   ▪ and not degrading under a given contrast threshold the local image contrasts of said identified specific bone(s) localization at different imaging positions along said vertical scanning direction, for all or part of patient thicknesses along said vertical scanning direction, for the frontal and/or lateral image(s),

○ or said both driving current intensity and voltage intensity modulations of said frontal and/or lateral radiation source(s) are performed simultaneously, preferably synchronously, and automatically, so as to improve a compromise between:

   ▪ lowering the global radiation dose received by a patient during said vertical scanning,
   ▪ and increasing, the local image contrasts of said identified specific bone(s) localization at different imaging positions along said vertical scanning direction, with respect to local image contrasts of said identified specific bone(s) localization at different imaging positions along said vertical scanning direction with same global radiation dose but without any driving current intensity modulation nor any driving voltage intensity modulation, for all or part of patient thicknesses along said vertical scanning direction, for the frontal and/or lateral image(s).

This means that it is thereby possible:

-     either to go toward the lowest possible global radiation dose while keeping correct predetermined image quality,
-     or to improve image quality, while not raising too much the global radiation dose,
-     or even, that the margin given by the invention can be distributed, partly in order to lower the radiation dose, partly in order to keep a predetermined image quality.

Preferably,

○ either said driving current intensity modulation(s) of said frontal and/or lateral radiation source(s), with no voltage intensity modulation of said frontal and/or lateral radiation source(s), is performed automatically, so as to improve a compromise between:

   ▪ lowering the global radiation dose received by a patient during said vertical scanning,
   ▪ and improving the contrast to noise ratio or the ratio between contrast to noise ratio and square root of said global radiation dose of said identified specific bone(s) localization at different imaging positions along said vertical scanning direction, with respect to local image contrasts of said identified specific bone(s) localization at different imaging positions along said vertical scanning direction with same global radiation dose but without any driving current intensity modulation, for all or part of patient thicknesses along said vertical scanning direction, for the frontal and/or lateral image(s),

○ or said both driving current intensity and voltage intensity modulations of said frontal and/or lateral radiation source(s) are performed simultaneously, preferably synchronously, and automatically, so as to improve a compromise between:

   ▪ lowering the global radiation dose received by a patient during said vertical scanning,

- and improving the contrast to noise ratio or the ratio between contrast to noise ratio and square root of said global radiation dose of said identified specific bone(s) localization at different imaging positions along said vertical scanning direction, with respect to local image contrasts of said identified specific bone(s) localization at different imaging positions along said vertical scanning direction with same global radiation dose but without any driving current intensity modulation nor any driving voltage intensity modulation, for all or part of patient thicknesses along said vertical scanning direction, for the frontal and/or lateral image(s).

This means that it is thereby possible:

- either to go toward the lowest possible global radiation dose while keeping a predetermined good image quality,
- or to improve deeply image quality, while not raising the global radiation dose,
- or even, that the margin given by the invention can be distributed, partly in order to lower the radiation dose, partly in order to keep a better predetermined image quality.

Preferably,

➢ said frontal multi-energy scout view acquisition is performed with at least 2 energy bins, or with at least 3 energy bins, or with at least 6 energy bins,

  ◦ and/or at most 20 energy bins, or at most 15 energy bins, or at most 10 energy bins,

➢ and/or said lateral multi-energy scout view acquisition is performed with at least 2 energy bins, or with at least 3 energy bins, or with at least 6 energy bins,

  ◦ and/or at most 20 energy bins, or at most 15 energy bins, or at most 10 energy bins.

Hence, the higher the number of bins, the more accurately different tissue textures within the patient body can be distinguished from one another, but at the cost of an increasing complexity of the system, and with the risk that less useful signal becomes available for each bin.

Preferably,

➢ said first and second frontal scout views are processed to a multi-material decomposition with at least two material thickness vertical profiles,

  ◦ preferably, either a bi-material decomposition between Al and PMMA, or a bi-material decomposition between HA (Hydroxyapatite) and $H_2O$,

➢ and/or said first and second lateral scout views are processed to a multi-material decomposition with at least two material thickness vertical vectors,

  ◦ preferably, either a bi-material decomposition between Al and PMMA, or a bi-material decomposition between HA and $H_2O$,

Indeed, on one side Al or HA present X-ray attenuation properties close to human bone, whereas on the other side, PMMA or $H_2O$ present X-ray attenuation properties close to human soft tissue.

Preferably,

➢ for each said radiation detector:

  ◦ a radiation detector pixel size ranges from $50\mu m$ to $250\mu m$, or ranges from $80\mu m$ to $150\mu m$, or is about $100\mu m$,
  ◦ and/or the total height of radiation detector ranges from 0.1cm to 1.2cm, or from 0.2cm to 1.0cm, or from 0.3cm to 0.7cm,
  ◦ and/or the total width of radiation detector ranges from 10cm to 80cm, or from 20cm to 70cm, or from 30cm to 60cm,
  ◦ and/or, said radiation detector can work in a Time Delay Summation mode.

Hence, the resolution of the image will be better, without creating too many artefacts, and the total useful width of the patient can be encompassed.

Preferably, said identified specific bone(s) localization includes a patient spine, preferably is a patient spine.

Indeed, patient spine is the specific bone(s) localization which is the most interesting to analyze in detail within a patient body, for orthopedic imaging purposes; therefore it is used to drive current intensity modulation.

Alternatively, the specific bone(s) localization may also be a pelvis or an arm or a leg of a standing patient along a vertical scanning direction, depending on the region of interest within the part of patient body which is imaged.

Preferably, said both driving current intensity and voltage intensity modulations of said frontal and/or lateral radiation source(s) are performed also so as to reach a value of signal to noise ratio which is constant and common to most of said imaging positions along said vertical scanning direction, preferably to all said imaging positions along said vertical scanning direction, for said frontal image and/or for said lateral image, but which can take two different values respectively for frontal image and for lateral image.

Preferably, for each of said frontal and/or lateral images, said signal to noise ratio value is constant and predetermined for each different patient organ to be imaged.

Preferably,

➢ for a frontal image of a patient spine, said standard signal to noise ratio value corresponds to a number of X-ray photons received per detector pixel comprised between 50 and 70, the radiological imaging method operator preferably having the possibility to deviate, via a manual command, from this standard value by at least -25% or +100%, more preferably by at least -50% or +200%,

➢ and/or for a lateral image of a patient spine, said standard signal to noise ratio value corresponds to a number of X-ray photons received per detector pixel comprised between 20 and 40, the radiological imaging method operator preferably having the possibility to deviate, via a manual command, from this standard value by at least -25% or +100%, more preferably by at least -50% or +200%.

Hence, with a constant and optimized signal to noise ratio along, or even all along, said vertical scanning direction, the local image contrasts of the identified specific bone(s) localization at different imaging positions along said vertical scanning direction are much improved, especially for what was indeed the region of interest within the frontal and/or lateral images.

Preferably, said frontal and/or lateral image, after having undergone at least said local image contrast improvements, is normalized by homogenization of raw radiations, in order to get rid of image artefacts coming from said driving current intensity and voltage intensity modulations, and preferably wherein said frontal and/or lateral image, after having been normalized, undergoes a contrast enhancement step.

Indeed, because of this driving modulation, there were some artefacts in the frontal and/or lateral images, which were superimposing some modulation patterns of clear and dark grey levels on the image, rendering those images a bit less comfortable to interpret for the radiological imaging method operator or practitioner.

Preferably, said identified specific bone(s) localization excludes metallic parts, if any, as for example metallic prosthesis of part of skeleton of patient body or as for example metallic protections put in place on patient body before performing said radiological imaging method.

Indeed, these foreign (to patient body) objects introduced within or on patient body, since being metallic and therefore stopping much more radiation (X-ray), than the rest of patient body, can lead to some bad optimization of the emitted dose, risking thereby to lead, for the altitudes corresponding to these foreign objects, to some over exposure to emitted radiation. Where driving voltage intensity is constant, if metal outliers are not excluded, consequences can be worse since more or all parameters are chosen for a maximal thickness, leading to emitting a radiation dose higher or much higher than needed, that would be detrimental to patient.

Preferably,

➢ modulations of both current intensity and voltage intensity:

∘ simultaneously increase both current intensity and voltage intensity for bigger patient thicknesses,
∘ simultaneously decrease both current intensity and voltage intensity for smaller patient thicknesses,
∘ current intensity variation rate being slower than voltage intensity variation rate.

Hence, all parts of patient body can be fully optimized, with respect to the compromise between the global radiation dose (preferably the lowest possible) and the image quality (preferably the highest possible).

Preferably, said current intensity modulation is maximized so as to also maximize said vertical scanning speed at a constant value.

Hence, for a given emitted radiation dose, so for a given radiation dose received by standing patient during said vertical scanning, both kept at same level, the total vertical scanning time is notably reduced, having the advantage of lowering the possibility for the standing patient to move and the effects of a patient motion, thereby reducing somewhat the risk of blurring and the risk of deformation of the frontal and lateral images.

Preferably, said operating mode can be either switched on or switched off manually by a radiological imaging method operator.

Hence, this very advantageous way of operating a radiological imaging apparatus is available, whereas it can be cancelled if and when the operator of this radiological imaging apparatus wants to get rid of it, in order for instance to fully manually operate this radiological imaging apparatus. The radiological imaging method according to advantageous embodiments of the invention presents 3 operating modes: a full manual mode, an AEC mode (AEC = Automatic Exposure Control) without modulation, an AEC mode with modulation.

Preferably, said operating mode can be used for patient morphotypes ranging from children to obese adults, and is dedicated to vertical scanning of large and/or obese patients, and/or wherein said operating mode is dedicated to vertical scanning of children patients.

The radiological imaging method according to the invention is all the more interesting when the thickness of the patient can be especially lower or especially higher than for an averaged size patient. This shows the capability of the radiological imaging method according to the invention to be very patient specific and account for wide attenuation ranges in long axis imaging. Of course, the radiological imaging method according to the invention works also very well for standard sized patients.

Preferably, said current intensity modulation(s) rate do(es) not go beyond a predetermined threshold of 5 mA per millisecond, or do(es) not go beyond a predetermined threshold of 2 mA per millisecond, or do(es) not go beyond a predetermined threshold of 1 mA per millisecond.

Hence, the radiological imaging method according to the invention can be performed also with relatively simple and cheap radiation sources with relatively slow current intensity driving capabilities.

Preferably, said current intensity modulation(s) at least range(s) from 20mA to 300mA, and preferably from 10mA to 400mA.

Hence, the radiological imaging method according to the invention can be performed also with relatively simple and cheap radiation sources with relatively limited ranges of current intensity driving capabilities.

Preferably, said voltage intensity modulation(s) at least range(s) from 60kV to 100kV, and preferably from 50kV to 130kV.

Hence, the radiological imaging method according to the invention can be performed also with relatively simple and cheap radiation sources with relatively limited extent of ranges of voltage intensity driving capabilities, while at the same time fully taking advantage of available ranges of voltage intensity driving capabilities.

Preferably, said vertical scanning speed value at least range(s) from 8cm/second to 20cm/second, and preferably from 0.4cm/second to 35cm/second.

Hence, the radiological imaging method according to the invention can be performed also with relatively simple and cheap radiation sources with relatively limited extent of ranges of vertical scanning speed capabilities, while at the same time fully taking advantage of available ranges of vertical scanning speed capabilities.

Preferably, each of said frontal and/or lateral scout view(s) is made by performing a preliminary vertical scanning of a standing patient along a vertical scanning direction with a reduced global radiation dose as compared to each of said frontal and lateral images, before making each of said frontal and lateral images, and preferably wherein said reduced global radiation is less than 10% of said global radiation dose, more preferably less than 5% of said global radiation dose.

Hence, depending on the thickness profile(s) and on the specific bone(s) localization of patient standing body along the vertical scanning direction, the modulation of driving current intensity, as well as possibly of vertical scanning speed, can be determined just before performing the vertical scanning which will result in effective frontal and lateral images of standing patient body performed with a limited but full radiation dose sufficient to make high quality frontal and lateral images. The scout view(s) can be performed at the cost of quite a limited over exposure to emitted radiation. The benefit can even be double: not only is the over exposure during scout view performance (+10% or +5%) very limited, but also it is very efficient to optimize compromise between global radiation dose received and enhancement of image contrast.

Preferably, pixels in said scout view are gathered together, preferably by zones of NxN pixels, more preferably by zones ranging from 2x2 pixels to 10x10 pixels, to make imaged zones.

Hence, image quality and image contrast are enhanced for the scout view, despite the very low level of emitted radiation dose used to perform this scout view.

Preferably, said images or said imaged zones are processed to identify salient points which in turn are used to compute said thickness profile(s) and to identify said specific bone(s) localization of a standing patient along said vertical scanning direction.

Hence, it is easier and more efficient to compute said thickness profile(s) and to identify said specific bone(s) localization of a standing patient along the vertical scanning direction, from the scout view, despite the very low level of emitted radiation dose.

Preferably, said images or said imaged zones are processed by a neural network to compute said thickness profile(s) and to identify said specific bone(s) localization of a standing patient along said vertical scanning direction.

Hence, it is easier and more efficient to identify said specific bone(s) localization of a standing patient along the vertical scanning direction, from the scout view, despite the very low level of emitted radiation dose.

Preferably, said 2 radiation sources slide vertically so as to perform vertical scanning of a pelvis or of a spine or of a full body of a standing patient along a vertical scanning direction.

Preferably, said 2 radiation detectors are respectively associated with said 2 radiations sources, said 2 radiation detectors being 2 Photon Counting Detectors (PCD) each being associated to an automatic image processing function automatically balancing image gray levels whatever radiation dose received on the sensitive surface of said radiation detector to homogenize responses of said detectors.

This is an interesting function since it would be harder for the radiological imaging method operator to correctly assess manually over exposure or under exposure to radiation signal emitted by radiation sources. Besides, Photon Counting Detectors present improved linearity and signal to noise ratio with respect to X-ray flux, as compared to gaseous detectors.

Preferably, said 2 radiation detectors are respectively associated with said 2 radiations sources, said 2 radiation detectors being 2 multi-energy counting detectors, preferably being 2 Energy Resolved Photon Counting Detectors (ERPCD).

Preferably, radiation is X-ray.

A standing patient or a patient in a standing position is a patient who is in a weight bearing position, contrary to a lying patient or to a patient who is in a lying position as in computed tomography. Another patient weight bearing position, alternative to patient standing position could be a patient seating position.

Preferably, said second energy threshold is chosen so as to improve image contrast more for lower patient thicknesses regions along vertical direction than for higher patient thicknesses regions along vertical direction, preferably said second energy threshold being chosen between 50keV and 90keV, preferably between 60keV and 80keV, more preferably said second energy threshold being chosen at 70keV.

Hence, even if wide patient chests can still be seen accurately, narrow arms or legs will not at all be sacrificed by being not saturated or very slightly saturated, i.e. over exposed due to system limitations.

Preferably, said first energy threshold and/or said second energy threshold are modified, and/or an associated spectral filtration, preferably k-edge filtration, is used and tuned, depending on said patient bone thickness and/or on said patient soft tissue thickness and/or on said patient specific bone localization at different imaging positions along said vertical scanning direction.

Hence, accuracy of end-user image used by practitioner is even improved.

Preferably,

➢ said frontal image and/or said lateral image are both mono-energy images, performed with said voltage intensity modulation(s) of said frontal radiation source and/or of said lateral radiation source,
➢ or said frontal image and/or said lateral image are both multi-energy images, performed with no voltage intensity modulation, of said frontal radiation source and/or of said lateral radiation source.

Hence, in order to get good image quality, with reduced global radiation dose, either the detector recording the image is simpler but the modulation is more complex, or the detector recording the image is more sophisticated but the modulation is simpler.

Advantageously, the multi-energy scout view is a dual-energy scout view.

To all preceding objects of the invention and/or to all preceding combinations, can be added either a mechanical cross scattering correction with vertical gap, as described for example in patent applications EP 16711889 or US 1607660, and/or a software cross scattering correction, as described for example in patent applications EP 17758269 or US 16628410, all of them being owned by same applicant.

To all preceding objects of the invention and/or to all preceding combinations, can be added and/or adapted some usual signal processing, for instance either signal pre-processing or signal post-processing as disclosed in patent application WO2021/094806 or in patent application WO2021/094404, owned by same applicant.

The present invention aims at providing a solution to provide an AEC system to a scanning stereo-radiographic system, this AEC being compliant to the IEC 62494-1 standard.

PMMA is Poly(methyl methacrylate) which is a thermoplastic often used as a glass substitute. Al is Aluminum.

Further features and advantages of the invention will appear from the following description of embodiments of the invention, given as non-limiting examples, with reference to the accompanying drawings listed hereunder.

BRIEF DESCRIPTION OF THE DRAWINGS

EP 4 525 724 B1

Fig. 1 shows an example of an imaging workflow of the radiological imaging method according to an embodiment of the invention, with current modulation but without voltage modulation.

Fig. 2 shows another example of an imaging workflow of the radiological imaging method according to an embodiment of the invention, both with current modulation and with voltage modulation.

Fig. 3 shows an example of multi-energy scout view acquisition step within the radiological imaging method according to an embodiment of the invention.

Fig. 4 shows another example of multi-energy scout view acquisition step within the radiological imaging method according to an embodiment of the invention.

Fig. 5 shows an example of bone localization computation step with profile of interest extraction within the radiological imaging method according to an embodiment of the invention.

Fig. 6 shows an example of a part of patient bone and soft tissue thickness computation step within the radiological imaging method according to an embodiment of the invention.

Fig. 7 shows an example of another part of patient bone and soft tissue thickness computation step within the radiological imaging method according to an embodiment of the invention.

Fig. 8 shows the interrelation between the total thickness, the PMMA thickness and the Al thickness.

Fig. 9 shows an example of a CNRD computation step over search space within the radiological imaging method according to an embodiment of the invention.

Fig. 10 shows an example of an optimal kV mapping step within the radiological imaging method according to an embodiment of the invention.

Fig. 11 shows an example of a normalization step within the radiological imaging method according to an embodiment of the invention, showing a patient frontal unnormalized image.

Fig. 12 shows an example of a normalization step within the radiological imaging method according to an embodiment of the invention, showing a patient frontal raw radiations image.

Fig. 13 shows an example of a normalization step within the radiological imaging method according to an embodiment of the invention, showing a patient frontal normalized image.

Fig. 14 shows an example of a normalization step within the radiological imaging method according to an embodiment of the invention, showing a patient lateral unnormalized image.

Fig. 15 shows an example of a normalization step within the radiological imaging method according to an embodiment of the invention, showing a patient lateral raw radiations image.

Fig. 16 shows an example of a normalization step within the radiological imaging method according to an embodiment of the invention, showing a patient lateral normalized image.

Fig. 17 shows an example of a mono-energy scout view with a signal to PMMA mapping only got by a radiological imaging method which is not according to an embodiment of the invention.

Fig. 18 shows an example of a multi-energy scout view with signal to PMMA and signal to Al mappings got by a radiological imaging method which is according to an embodiment of the invention.

Fig. 19 shows an example of simulation of expected tube power profiles, with both frontal and lateral mono-energy scout views, not according to an embodiment of the invention, with current modulation but without voltage modulation.

Fig. 20 shows an example of current modulation profile corresponding to figure 19.

Fig. 21 shows an example of voltage fixed value corresponding to figure 19.

Fig. 22 shows an example of frontal bone localization profile along patient profile of interest corresponding to figure 19.

Fig. 23 shows an example of lateral bone localization profile along patient profile of interest corresponding to figure 19.

Fig. 24 shows an example of simulation of expected tube power profiles, with both frontal and lateral mono-energy scout views, not according to an embodiment of the invention, both with current modulation and with voltage modulation.

Fig. 25 shows an example of current modulation profile corresponding to figure 24.

Fig. 26 shows an example of voltage modulation profile corresponding to figure 24.

Fig. 27 shows an example of frontal bone localization profile along patient profile of interest corresponding to figure 24.

Fig. 28 shows an example of lateral bone localization profile along patient profile of interest corresponding to figure 24.

Fig. 29 shows an example of simulation of expected tube power profiles, with both frontal and lateral multi-energy scout views, according to an embodiment of the invention, both with current modulation and with voltage modulation.

Fig. 30 shows an example of current modulation profile corresponding to figure 29.

Fig. 31 shows an example of voltage modulation profile corresponding to figure 29.

Fig. 32 shows an example of frontal bone localization profile along patient profile of interest corresponding to figure 29.

Fig. 33 shows an example of lateral bone localization profile along patient profile of interest corresponding to figure 29.

Fig. 34 shows an example of frontal bone localization profile along patient profile of interest in a modulated image obtained through simulation.

Fig. 35 shows an example of lateral bone localization profile along patient profile of interest in a modulated image obtained through simulation.

Fig. 36 shows an example of simulation of frontal signal profile along patient profile of interest.

Fig. 37 shows an example of simulation of frontal deviation index profile along patient profile of interest.

Fig. 38 shows an example of simulation of lateral signal profile along patient profile of interest.

Fig. 39 shows an example of simulation of lateral deviation index profile along patient profile of interest.

Fig. 40 shows an example of structure of imaging device to implement the radiological imaging method according to an embodiment of the invention.

Fig. 41 shows an example of region of detector used for mean value computation during implementation of the radiological imaging method according to an embodiment of the invention.

Fig. 42 shows an example of optimal kV mapping during the radiological imaging method not according to an embodiment of the invention, the scout view being mono-energy.

Fig. 43 shows an example of optimal kV mapping during the radiological imaging method according to an embodiment of the invention, the scout view being multi-energy.

Fig. 44 shows an example of deviation index map for an optimal kV mapping during the radiological imaging method not according to an embodiment of the invention, the scout view being mono-energy.

Fig. 45 shows an example of deviation index map for an optimal kV mapping during the radiological imaging method according to an embodiment of the invention, the scout view being multi-energy.

## DETAILED DESCRIPTION OF THE INVENTION

In the following description, without mention to the contrary, what is said from frontal scout view or image can be applied similarly respectively to lateral scout view or image, and vice-versa. All what is done both for frontal and lateral scout view can be done either for frontal scout view only or for lateral scout view only, if only frontal scout view or only lateral scout view is of interest to practitioner or to patient. All what is done both for frontal and lateral image can be done either for frontal image only or for lateral image only, if only frontal image or only lateral image is of interest to practitioner or patient. Wherever a profile is mentioned, except mention to the contrary, a sequence of several partial or regional mean values (a mean value per zone or organ, for instance, leg, pelvis, spine, neck, head, or portions of those) or sometimes a single mean value may be sufficient, although results will be less accurate.

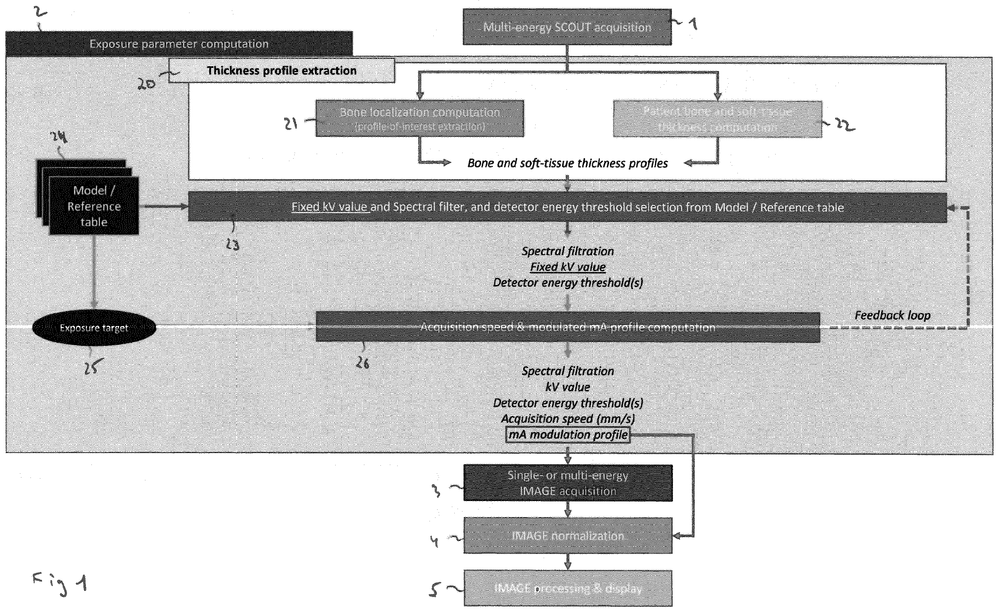

Fig. 1 shows an example of a imaging workflow of the radiological imaging method according to an embodiment of the invention, with current modulation but without voltage modulation.

First, a step 1 of acquisition of a frontal scout view and a lateral scout view is performed.

Then, a step 20 of thickness profile extraction is performed from the frontal scout view and the lateral scout view performed at acquisition step 1. This step 20 includes a sub-step 21 of bone localization computation with a profile of interest extraction along patient height performed in parallel and simultaneously to a sub-step 22 of computation of both a patient bone thickness and of a patient soft tissue thickness, then both a patient bone thickness profile and of a patient soft tissue thickness profile along patient height are processed by extraction of respective thicknesses values along the profile of interest coordinates. In sub-step 21, coordinates {x_i} (x_1, x_2... x_N), corresponding to localization of points in the profile of interest, are extracted from the scout views. In sub-step 22, conversions between signals and thicknesses are performed, which result in a bone thickness image fBone and in a soft tissue thickness image fSoft. Combined result of sub-steps 21 and 22 is the collection of {fBone(x_i)} and of {fSoft(x_i)}.

At the end of step thickness profile extraction 20, both a patient bone thickness profile and of a patient soft tissue thickness profile along patient height are available for next step which is a determination and selection step 23.

Then, a step 23 of determination of fixed voltage value (in kV) and of spectral filtration, and also of selection of detector energy threshold(s), from a model and/or reference table 24 which includes a catalog of models and/or of references, based on the patient bone thickness profile and on a patient soft tissue thickness profile along patient height. The model and/or reference table 24 gives an exposure target 25 which corresponds to the model or to the reference which has been selected in determination and selection step 23. At the end of determination and selection step 23, a spectral filtration, a fixed voltage value, one or more detector energy threshold(s) are available for next computation step 26.

Then, a computation step 26 computes an acquisition speed for the vertical scanning along the height of the standing (or seating) patient and of a current modulation profile along patient height. Computation step 26 also uses the exposure target 25. Computation step also performs a feedback loop toward determination and selection step 23. At the end of computation step 26, a spectral filtration, a fixed voltage value, one or more detector energy threshold(s), a vertical scanning acquisition speed (in mm per second), a current modulation profile in mA along patient height, are available for next image acquisition step 3. The current modulation profile along patient height will also be directly available for the image normalization step 4.

All steps 20, 23, 24, 25, 26 are part of an exposure parameter computation routine 2.

Then, after this exposure parameter computation routine 2 has been fully completed, an image acquisition step 3 is performed, based on spectral filtration, fixed voltage value, one or more detector energy threshold(s), vertical scanning acquisition speed (in mm per second), current modulation profile (in mA) along patient height. This acquired image can be a mono-energy image or can be a multi-energy image. Preferably, this acquired image is a multi-energy image. The acquired image can include a frontal image and/or a lateral image. The acquired image includes preferably a frontal image and a lateral image.

The frontal and lateral acquired images are then normalized in a normalization step 4.

The frontal and lateral normalized images can then be further processed (with post-processing steps) and/or afterwards displayed on a screen to be seen by the practitioner, in a processing and display step 5.

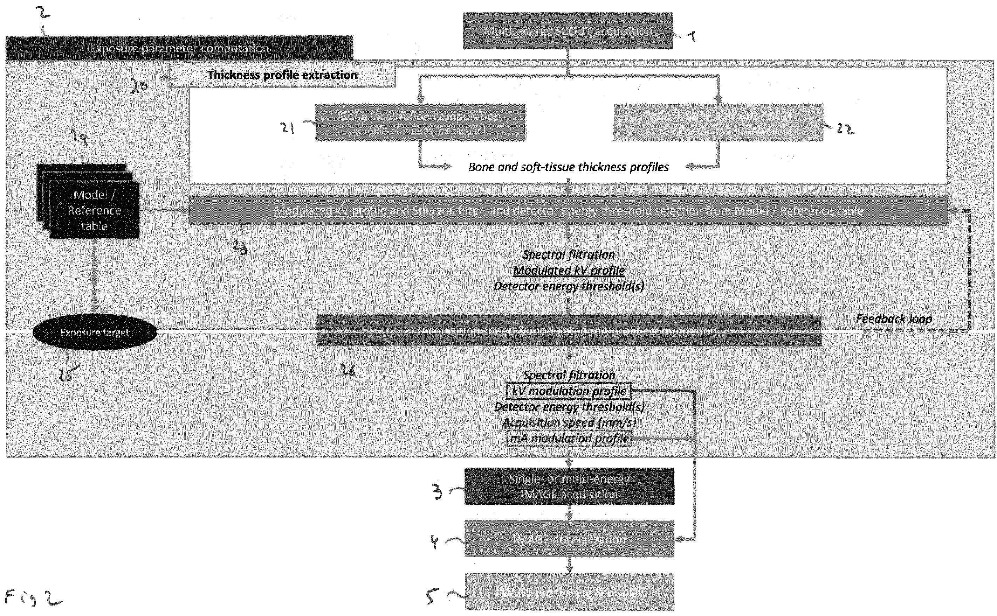

Fig. 2 shows another example of a imaging workflow of the radiological imaging method according to an embodiment of the invention, both with current modulation and with voltage modulation.

First, a step 1 of acquisition of a frontal scout view and a lateral scout view is performed.

Then, a step 20 of thickness profile extraction is performed from the frontal scout view and the lateral scout view performed at acquisition step 21. This step 20 includes a sub-step 21 of bone localization computation with a profile of interest extraction along patient height performed in parallel and simultaneously to a sub-step 22 of computation of both a patient bone thickness and of a patient soft tissue thickness, then both a patient bone thickness profile and of a patient soft tissue thickness profile along patient height are processed by extraction of respective thicknesses values along the profile of interest coordinates. At the end of step thickness profile extraction 20, both a patient bone thickness profile and of a patient soft tissue thickness profile along patient height are available for next step which is a determination and selection step 23.

Then, a step 23 of determination of a voltage modulation profile (in kV = kilovolts) along patient height and of spectral filtration, and also of selection of detector energy threshold(s), from a model and/or reference table 24 which includes a catalog of models and/or of references, based on the patient bone thickness profile and on a patient soft tissue thickness profile along patient height. The model and/or reference table 24 gives an exposure target 25 which corresponds to the model or to the reference which has been selected in determination and selection step 23. At the end of determination and selection step 23, a spectral filtration, a voltage modulation profile along patient height, one or more detector energy threshold(s) are available for next computation step 26.

Then, a computation step 26 computes of an acquisition speed for the vertical scanning along the height of the standing (or seating) patient and of a current modulation profile along patient height. Computation step 26 also uses the exposure target 25. Computation step also performs a feedback loop toward determination and selection step 23. At the end of computation step 26, a spectral filtration, a voltage modulation profile along patient height, one or more detector energy threshold(s), a vertical scanning acquisition speed (in mm per second), a current modulation profile (in mA) along patient height. The voltage modulation profile along patient height and the current modulation profile along patient height will also be directly available for the image normalization step 4, are available for next image acquisition step 3.

All steps 20, 23, 24, 25, 26 are part of an exposure parameter computation routine 2.

Then, after this exposure parameter computation routine 2 has been fully completed, an image acquisition step 3 is performed, based on spectral filtration, voltage modulation profile (in kV) along patient height, one or more detector energy threshold(s), vertical scanning acquisition speed (in mm per second), current modulation profile (in mA) along patient height. This acquired image can be a mono-energy image or can be a multi-energy image. Preferably, this acquired image is a multi-energy image, but can also be a mono-energy image. The acquired image can include a frontal image and/or a lateral image. The acquired image includes preferably a frontal image and a lateral image.

The frontal and lateral acquired images are then normalized in a normalization step 4.

The frontal and lateral normalized images can then be further processed (with post-processing steps) and/or afterwards displayed on a screen to be seen by the practitioner, in a processing and display step 5.

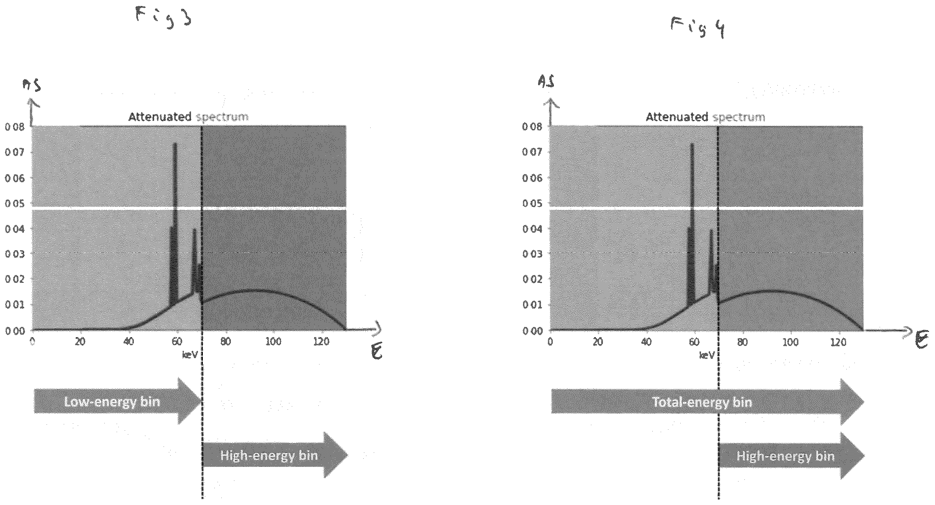

Fig. 3 shows an example of multi-energy scout view acquisition step within the radiological imaging method according to an embodiment of the invention.

A normalized attenuated spectrum AS is represented as a function of an energy E expressed in keV.

In multi-energy scout view mode, the detector reads low-energy and high-energy scout views directly, and deduces the total-energy scout view by adding together the high-energy scout view and the low-energy scout view.

In mono-energy or single-energy scout view mode, there would be only a single bin which would correspond to the total energy bin.

Fig. 4 shows another example of multi-energy scout view acquisition step within the radiological imaging method according to an embodiment of the invention.

A normalized attenuated spectrum AS is represented as a function of an energy E expressed in keV.

In multi-energy scout view mode, the detector reads a total-energy scout view and a high-energy scout view, and deduces the low-energy scout view by subtracting the high-energy scout view from the total-energy scout view.

In mono-energy or single-energy scout view mode, there would be only a single bin which would correspond to the total energy bin.

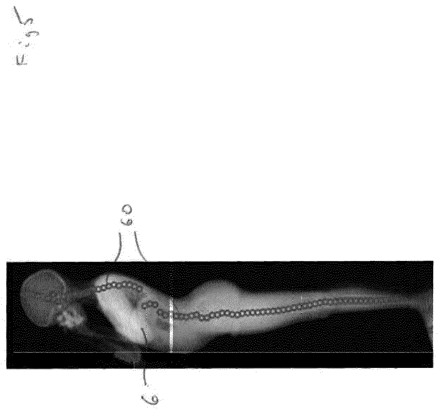

Fig. 5 shows an example of bone localization computation step with profile of interest extraction within the radiological imaging method according to an embodiment of the invention.

In figure 5, there is a scout view 6, here a lateral scout view 6, with salient points 60 located along the lateral image 6 of the patient skeleton, the salient points 60 representing the patient specific bone localization at different imaging positions along said vertical scanning direction which is also standing patient height here.

These salient points 60 represent a curve which has been extracted from the patient lateral image 6 by summarizing the detector signal values in anatomical landmarks with clinical interest along the slot scan. Therefore, several processing techniques can be used, which can be based on image filtering and segmentation techniques, or on landmark extraction from machine learning techniques.

This is a way to perform the sub-step 21 of bone localization computation.

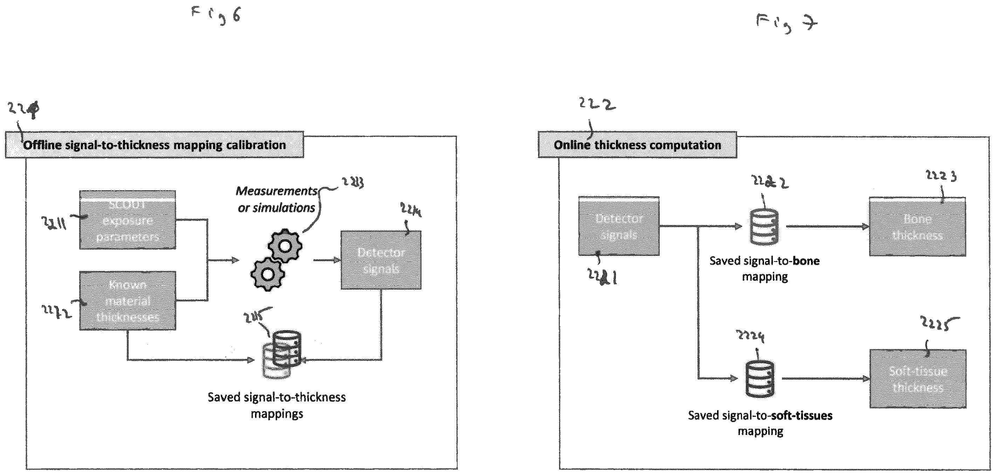

Fig. 6 shows an example of a part of patient bone and soft tissue thickness computation step within the radiological imaging method according to an embodiment of the invention.

The sub-step 22 of computation of both a patient bone thickness and of a soft tissue thickness is performed each time by an online operation 222 of online thickness computation 222 on figure 7 using information got separately offline by an offline operation 221 of offline signal-to-thickness mapping calibration on figure 6.

In the offline operation 221 of offline signal-to-thickness mapping calibration, scout exposure parameters 2211 and known material thicknesses 2212 are used as inputs to a measurement or simulation 2213 which gives as output measured or simulated detector signals 2214. Then, both known material thicknesses 2212 and measured or simulated detector signals 2214 are saved and stored in a signal-to-thickness mappings database 2215. The signal-to-thickness mappings database 2215, which is computed offline, includes mappings from detector signal counts to corresponding material thicknesses. These mappings can be for example look-up tables (LUT) or model fits. Contrary to mono-energy scout view, where the mapping is based on the total-energy signal, the multi-energy scout view mapping, here the dual-energy scout view mapping is based on the signal pair, including both low-energy signal and high-energy signal, with computation of one mapping per material: soft tissue for low-energy signal, bone for high-energy signal. PMMA thicknesses are stored in a system of coordinates high energy and low energy. Al thicknesses are stored in a system of coordinates high energy and low energy.

Fig. 7 shows an example of another part of patient bone and soft tissue thickness computation step within the radiological imaging method according to an embodiment of the invention.

In the online operation 222 of online thickness computation, measured detector signals 2221 are used as inputs to both a sub-database 2222 storing signal-to-bone thickness mappings and to a sub-database 2224 storing signal-to-soft-tissue thickness mappings. The sub-database 2222 storing signal-to-bone thickness mappings gives as output a bone thickness profile 2223. The sub-database 2224 storing signal-to-soft-tissue thickness mappings gives as output a soft-tissue thickness profile 2225.

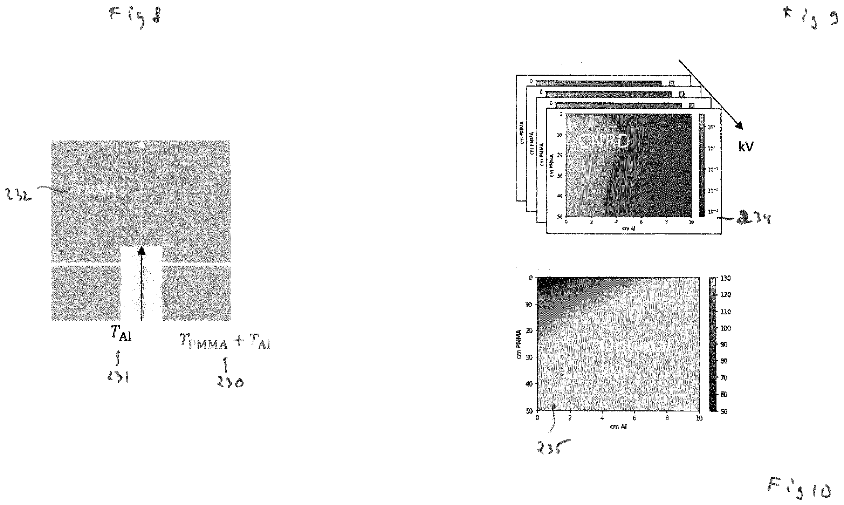

Fig. 8 shows the interrelation between the total thickness, the PMMA thickness and the Al thickness. In phantoms or avatars used to build up the signal-to-thickness mappings database 2215, including both a signal-to-bone sub-database 2222 and a soft-tissue mappings sub-database 2224, the total thickness $T_{PMMA+Al}$ 230 is equal to the sum of $T_{PMMA}$ 232 and of $T_{Al}$ 231. In imaged patient bodies and in corresponding images processed using the signal-to-thickness mappings database 2215, including both a signal-to-bone sub-database 2222 and a soft-tissue mappings sub-database 2224, the total thickness $T_{sort\ tissue\ +\ bone}$ 230 is equal to the sum of $T_{soft\ tissue}$ 232 and of $T_{bone}$ 231.

The step 23 of determination of a voltage modulation profile (in kV) along patient height and of spectral filtration, and also of selection of detector energy threshold(s), from a model and/or reference table 24 which includes a catalog of models and/or of references, based on the patient bone thickness profile and of a patient soft tissue thickness profile along patient height, is based on a mapping from material thicknesses to voltage values expressed in kV (kilovolts). This mapping may be given by empirically fine-tuned look-up-tables as a first alternative, or optimally determined according to a task metric as a second alternative.

In the second alternative, the task metric should preferably satisfy at best the following rules:

- be invariant with respect to the product tube current by exposure time, or at least be a monotone function of the product tube current by exposure time (expressed in mAs which are milliamps second),
- represent how good a task performs as a function of voltage and material thicknesses (the material thicknesses are either a one-dimensional vector, in the case of a single-energy scout view, or a multi-dimensional vector, in the case of a multi-energy scout view as in present invention): the better the task, the higher the metric,
- be meaningful throughout the slot scan, i.e., along the whole profile-of-interest.

This task metric is denoted $W(kV, \vec{p})$, where $\vec{p}$ is a vector of additional data-dependent parameters such as the single-material or multi-material thickness profiles, or tube spectral filtration options for the image acquisition. The optimal voltage kV selection amounts to choosing

$$kV(\vec{p}) = \underset{kV'}{\mathrm{argmax}}\{W(kV', \vec{p})\}$$

This mapping is computed offline and saved, for example either as a look-up table or as a model fit. Along with this mapping, the mapping of detector exposure targets (e.g., detector signals) per product tube current by exposure time (in mAs) $\vec{S}(kV, \vec{p})$ is also saved for example either as a look-up table or as a model fit. $\vec{S}(kV, \vec{p})$ is used in the task metric.

As an illustration, a specific example of task metric used for voltage kV optimization in the dual-energy scout view case, named the CNR-to-dose ratio (CNRD), is now described.

As an example, a two-material basis (PMMA, Al) provided by a dual-energy scout view, is considered.

The task is then twofold:

- maximize the local contrast resolution of the Al material surrounded by PMMA,
- minimize the entrance dose that is needed to achieve a unit detector signal value behind the (PMMA, Al) projection line (what corresponds to the ALARA principle).

The local contrast resolution can be measured by the contrast-to-noise ratio (CNR):

$$\text{CNR} = \frac{|B - C|}{\sqrt{B}}$$

$$B = \int_0^{kV} I_0(E) \cdot e^{-(T_{PMMA} + T_{Al}) \cdot \mu_{PMMA}(E)} \, dE$$

$$C = \int_0^{kV} I_0(E) \cdot e^{-(T_{PMMA} \cdot \mu_{PMMA}(E) + T_{Al} \cdot \mu_{Al}(E))} \, dE$$

The entrance dose can be monitored by using the spectrum air kerma value D

The CNRD is then defined as:

$$W(kV, T_{PMMA}, T_{Al}) = \text{CNRD} = \frac{\text{CNR}}{\sqrt{D}}$$

It is independent of the product tube current by exposure time.

Fig. 9 shows an example of a CNRD computation step over search space within the radiological imaging method according to an embodiment of the invention.

A database of CNRD maps 234 each being a function both of Al thickness and of PMMA thickness, for a specific voltage value, is searched.

Fig. 10 shows an example of an optimal kV mapping step within the radiological imaging method according to an embodiment of the invention.

A database of CNRD maps 234 has been searched, so as to get at an optimal voltage (kV) map 235, which is a map showing the best voltage to use as a function of both Al and PMMA thicknesses.

The optimization, shown on both figures 9 and 10, works as follows:

- explore the $(kV, T_{PMMA}, T_{Al})$ space offline, either by using simulations or by taking measurements on the system (or by using hybrid methods)

- deduce $\quad kV(T_{PMMA}, T_{Al}) = \underset{kV'}{\operatorname{argmax}}\{\text{CNRD}(kV', T_{PMMA}, T_{Al})\}$

It is possible to deduce the optimal voltage (in kV) values for a mono-energy scout view by computing $kV(T_{PMMA}, 0)$ (using data extrapolation methods) if the used equivalent thickness material is PMMA.

Here, only the kV-space (p = kV) at fixed spectral filtration and fixed detector energy threshold has been optimized, for reasons of simplicity. But, if optimization is performed on multi-valued parameters, e.g., p = (kV, Filter, Thresholds), the rationale remains the same :

- still explore the search space (p, $T_{PMMA}$, $T_{Al}$),

- and then compute $\quad p(T_{PMMA}, T_{Al}) = \underset{p'}{\operatorname{argmax}}\{\text{CNRD}(p', T_{PMMA}, T_{Al})\}$

The computation step 26 performs computation of an acquisition speed for the vertical scanning along the height of the standing (or seating) patient and of a current modulation profile along patient height.

The current profile and the vertical scan speed selection strategy, for a given voltage value or a given voltage profile, is similar to the strategy used in patent application WO2021/094806, in summary:

- computation of an exposure budget (the needed current value multiplied by the exposure time to reach the exposure target),
- maximization of the vertical scan speed, given the available tube current budget (constrained by hardware specifications in terms of min and max tube powers and min and max instantaneous current values),
- computation of the corresponding current profile therefrom.

Given an optimal voltage value for a set of parameters $\vec{p}$ (including thickness profiles), the product of current value by exposure time (in mAs : milliamperes seconds) that is required in order to reach target signal $S_T$ is

$$mAs = \frac{S_T}{\overline{S}(kV, \vec{p})}$$

The exposure time t is given by the highest current value that can be used for the given voltage value, when accounting for tube power limits and lower as well as upper current bounds. The acquisition speed is selected (as a free value or among a collection of admissible system speeds) as the smallest speed among the computed speeds along the thickness profiles. The current values are then updated to match the target signal, given this fixed acquisition speed.

Wherever the current values fall below, respectively beyond the current bounds, voltage may be decreased, respectively increased, in order to bring them back to the admissible range of values, until voltage values themselves reach their lower and/or upper bounds. Matching the target signal is preferred over keeping the optimal voltage value, this preference being met thanks to the feedback loop from this step 26 to step 23 (see figures 1 and 2).

Once the optimal exposure parameters are selected, their profiles along the slot scan may further be adapted to hardware constraints (e.g., on the slopes of the current and voltage profiles) if these constraints were not yet accounted for in the former steps. The exposure parameters are then formatted in files that are readable by the system and an image acquisition step 3 can be performed (see figures 1 and 2).

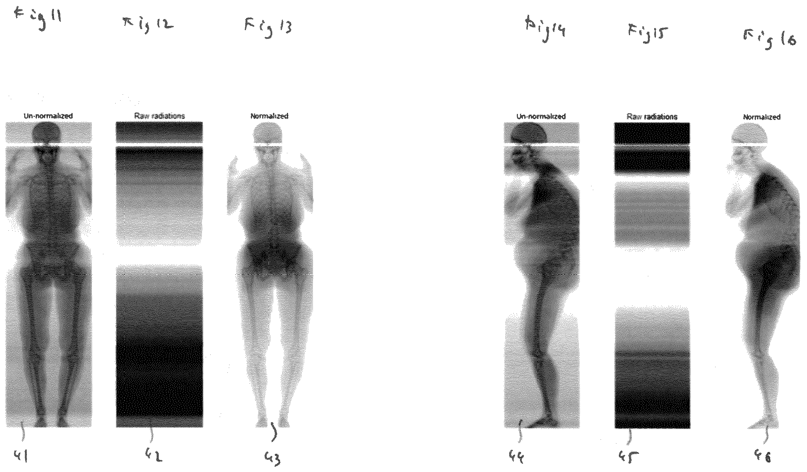

Figures 11 to 16 show the effect of a normalization step 4. An accurate data normalization step 4 (see figures 1 and 2) may include either an additional air measurement with the same exposure techniques (up to a factor on current values), or simulations, or hybrid methods combining the online image acquisition with simulations. Normalizing the image acquisition by the air measurement (also called "raw radiations") is enough, but this normalization step 4 may also advantageously include an image processing algorithm in order to account for system variabilities. Once the normalization step 4 is performed, the standard image processing pipeline may be used.

Fig. 11 shows an example of a normalization step within the radiological imaging method according to an embodiment of the invention, showing a patient frontal unnormalized image. There is a patient frontal unnormalized image 41.

Fig. 12 shows an example of a normalization step within the radiological imaging method according to an embodiment of the invention, showing a patient frontal raw radiations image. There is a frontal raw radiations image 42, which is the frontal image of air without patient.

Fig. 13 shows an example of a normalization step within the radiological imaging method according to an embodiment of the invention, showing a patient frontal normalized image. There is a patient frontal normalized image 43 which is obtained by dividing the patient frontal unnormalized image 41 by the frontal raw radiations image 42.

Fig. 14 shows an example of a normalization step within the radiological imaging method according to an embodiment of the invention, showing a patient lateral unnormalized image. There is a patient lateral unnormalized image 44.

Fig. 15 shows an example of a normalization step within the radiological imaging method according to an embodiment of the invention, showing a patient lateral raw radiations image. There is a lateral raw radiations image 45, which is the lateral image of air without patient.

Fig. 16 shows an example of a normalization step within the radiological imaging method according to an embodiment of the invention, showing a patient lateral normalized image. There is a patient lateral normalized image 46 which is obtained by dividing the patient lateral unnormalized image 44 by the lateral raw radiations image 45.

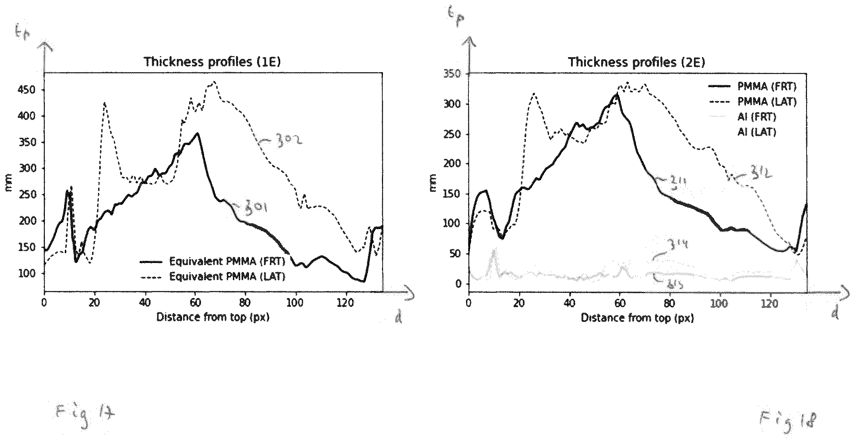

Figures 17 to 39 will now show simulation results for the radiological imaging method according to an embodiment of the invention.

A simulated voxelated anthropomorphic phantom made of PMMA and Al is used. Thickness profile(s) are extracted both in frontal and lateral scout views and used to compute the exposure parameters.

Simulated cases are:

- mono-energy scout view, leading to a tube current modulation:

  ◦ with fixed voltage,
  ◦ with modulated voltage,

- dual-energy scout view (as in present invention), leading to a tube current modulation with modulated voltage.

Exposure quality assessment is performed:

- the profile-of-interest is extracted (as explained in the bone localization computation sub-step 21 on figures 1 and 2), the signal profile being thus the smoothed image detector signal along this profile-of-interest, denoted S,

- the local deviation index profile is computed as $DI = 10 \log_{10}\left(\frac{S}{S_T}\right)$

- the global deviation index value is $DI_X = 10 \log_{10}\left(\frac{X(S)}{S_T}\right)$, where X(S) describes the central tendency of S along the profile-of-interest, such as the mean M(S) or the median m(S) following IEC 62494-1 standard. The closer to zero the deviation index, the better the exposure quality.

Simulation results will show that:

- the fixed voltage case lead to under-exposed areas where the bones are predominant with respect to the PMMA (patient neck and patient legs for example),
- the voltage modulation with PMMA-only thickness profiles brings back the signal profiles closer to their targets, but still miss the target in thinner areas,
- the voltage modulation with both PMMA and Al thickness profiles results in flatter signal profiles, with deviation indices closer to zero.

Fig. 17 shows an example of a mono-energy scout view with a signal to PMMA mapping only got by a radiological imaging method which is not according to an embodiment of the invention.

Curves 301 and 302 show the thickness profiles tp along the distance d from top of patient (summit of head) which corresponds to the patient height. Curve 301 shows the frontal soft tissue thickness profile along the patient height. Curve 302 shows the lateral soft tissue thickness profile along the patient height.

Fig. 18 shows an example of a multi-energy scout view with signal to PMMA and a signal to Al mappings got by a radiological imaging method which is according to an embodiment of the invention.

Curves 311, 312, 313 and 314, show the thickness profiles tp along the distance d from top of patient (summit of head) which is also the patient height (starting from top of patient). Curve 311 shows the frontal soft tissue thickness profile along the patient height. Curve 312 shows the lateral soft tissue thickness profile along the patient height. Curve 313 shows the frontal bone thickness profile along the patient height. Curve 314 shows the lateral bone thickness profile along the patient height.

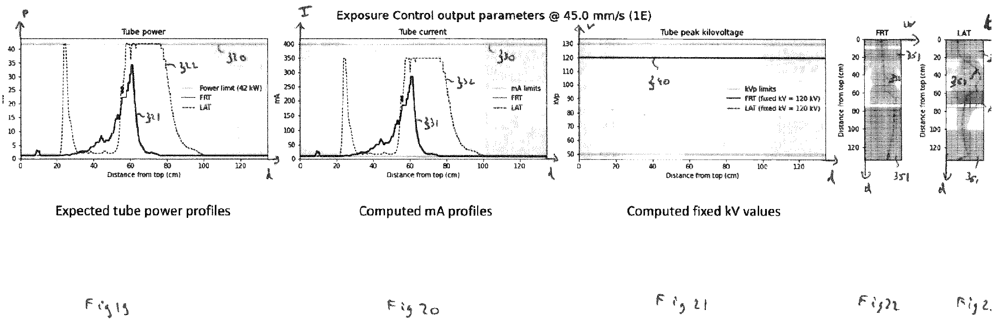

Fig. 19 shows an example of simulation of expected tube power profiles, with both frontal and lateral mono-energy scout views, not according to an embodiment of the invention, with current modulation but without voltage modulation. Vertical scanning speed is 45mm per second.

Curve 321 shows the frontal tube power profile P along the distance d from top of patient (summit of head). Curve 322 shows the lateral tube power profile P along the distance d from top of patient (summit of head). Horizontal line 320 shows the tube power limit which cannot be exceeded without saturation. Maximal tube power limit is 42 kW.

Fig. 20 shows an example of current modulation profile corresponding to figure 19. Vertical scanning speed is 45mm per second.

Curve 331 shows the frontal tube current profile I along the distance d from top of patient (summit of head). Curve 332 shows the lateral tube current profile I along the distance d from top of patient (summit of head). Horizontal line 330 shows the tube current limit which cannot be exceeded without saturation. Available tube current ranges from a minimum of 10mA to a maximum of 400mA.

Fig. 21 shows an example of voltage fixed value corresponding to figure 19. Vertical scanning speed is 45mm per second.

Horizontal line 340 shows the fixed tube voltage which is kept constant along the patient height. Available tube voltage ranges from a minimum of 50kV to a maximum of 130kV.

Fig. 22 shows an example of frontal bone localization profile along patient height corresponding to figure 19. Vertical scanning speed is 45mm per second.

Curve 350 shows the frontal bone localization along the distance d from top of patient (summit of head) and along the width w of the patient.

The grayed areas correspond to profile points that reach the system lower and/or upper limits, and they correspond to the grayed-out lined frontal zones 351 and lateral zones 361 respectively in the frontal and lateral images of figures 22 and 23 (the bone localization profiles 350 and 360 are shown as dashed lines). Here numerous and large frontal zones 351 can be seen.

Fig. 23 shows an example of lateral bone localization profile along patient profile of interest corresponding to figure 19. Vertical scanning speed is 45mm per second.

Curve 360 shows the lateral bone localization along the distance d from top of patient (summit of head) and along the thickness th of the patient.

The grayed areas correspond to profile points that reach the system lower and/or upper limits, and they correspond to the grayed-out lined frontal zones 351 and lateral zones 361 respectively in the frontal and lateral images of figures 22 and 23 (the bone localization profiles 350 and 360 are shown as dashed lines). Here numerous and large lateral zones 361 can be seen.

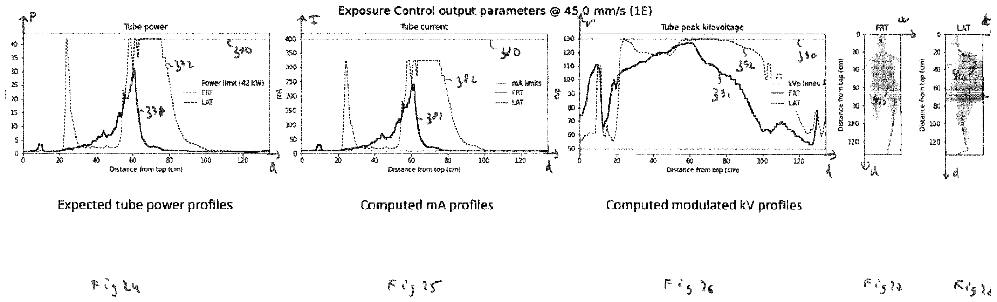

Fig. 24 shows an example of simulation of expected tube power profiles, with both frontal and lateral mono-energy scout views, not according to an embodiment of the invention, both with current modulation and with voltage modulation. Vertical scanning speed is 45mm per second.

Curve 371 shows the frontal tube power profile P along the distance d from top of patient (summit of head). Curve 372 shows the lateral tube power profile P along the distance d from top of patient (summit of head). Horizontal line 370 shows the tube power limit which cannot be exceeded without saturation. Maximal tube power limit is 42 kW.

Fig. 25 shows an example of current modulation profile corresponding to figure 24. Vertical scanning speed is 45mm per second.

Curve 381 shows the frontal tube current profile I along the distance d from top of patient (summit of head). Curve 382 shows the lateral tube current profile I along the distance d from top of patient (summit of head). Horizontal line 380 shows the tube current limit which cannot be exceeded without saturation. Available tube current ranges from a minimum of 10mA to a maximum of 400mA.

Fig. 26 shows an example of voltage modulation profile corresponding to figure 24. Vertical scanning speed is 45mm per second.

Curve 391 shows the frontal tube voltage profile V along the distance d from top of patient (summit of head). Curve 392 shows the lateral tube voltage profile V along the distance d from top of patient (summit of head). Horizontal line 390 shows the tube voltage limit which cannot be exceeded without saturation. Available tube voltage ranges from a minimum of 50kV to a maximum of 130kV.

Fig. 27 shows an example of frontal bone localization profile along patient profile of interest corresponding to figure 24. Vertical scanning speed is 45mm per second.

Curve 400 shows the frontal bone localization along the distance d from top of patient (summit of head) and along the width w of the patient.

The grayed areas correspond to profile points that reach the system lower and/or upper limits, and they correspond to the grayed-out lined lateral zones 411 in the lateral image of figure 28 (the bone localization profiles 400 and 410 are shown as dashed lines). Here no frontal zone can be seen.

Fig. 28 shows an example of lateral bone localization profile along patient profile of interest corresponding to figure 24. Vertical scanning speed is 45mm per second.

Curve 410 shows the lateral bone localization along the distance d from top of patient (summit of head) and along the thickness th of the patient.

The grayed areas correspond to profile points that reach the system lower and/or upper limits, and they correspond to the grayed-out lined lateral zones 411 in the lateral image of figure 28 (the bone localization profiles 400 and 410 are shown as dashed lines). Here, there is a unique but somewhat large lateral zone 411.

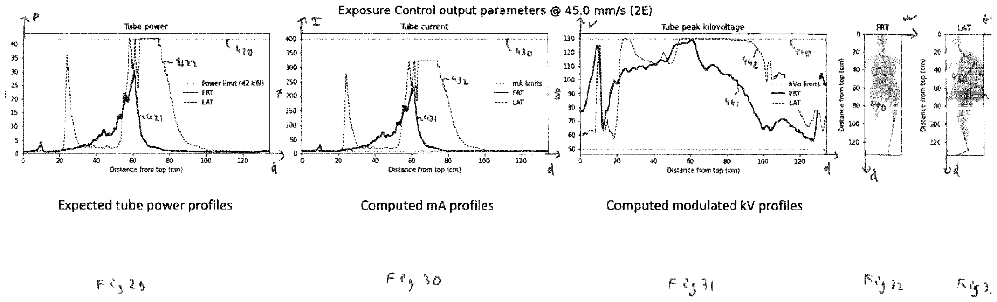

Fig. 29 shows an example of simulation of expected tube power profiles, with both frontal and lateral mono-energy scout views, according to an embodiment of the invention, both with current modulation and with voltage modulation. Vertical scanning speed is 45mm per second.

Curve 421 shows the frontal tube power profile P along the distance d from top of patient (summit of head). Curve 422 shows the lateral tube power profile P along the distance d from top of patient (summit of head). Horizontal line 420 shows the tube power limit which cannot be exceeded without saturation. Maximal tube power limit is 42 kW (kW = kilowatts).

Fig. 30 shows an example of current modulation profile corresponding to figure 29. Vertical scanning speed is 45mm (mm = millimeters) per second.

Curve 431 shows the frontal tube current profile I along the distance d from top of patient (summit of head). Curve 432 shows the lateral tube current profile I along the distance d from top of patient (summit of head). Horizontal line 430 shows the tube current limit which cannot be exceeded without saturation. Available tube current ranges from a minimum of 10mA to a maximum of 400mA.

Fig. 31 shows an example of voltage modulation profile corresponding to figure 29. Vertical scanning speed is 45mm per second.

Curve 441 shows the frontal tube voltage profile V along the distance d from top of patient (summit of head). Curve 442 shows the lateral tube voltage profile V along the distance d from top of patient (summit of head). Horizontal line 440 shows the tube voltage limit which cannot be exceeded without saturation. Available tube voltage ranges from a minimum of 50kV to a maximum of 130kV.

Fig. 32 shows an example of frontal bone localization profile along patient profile of interest corresponding to figure 29. Vertical scanning speed is 45mm per second.

Curve 450 shows the frontal bone localization along the distance d from top of patient (summit of head) and along the width w of the patient.

The grayed areas correspond to profile points that reach the system lower and/or upper limits, and they correspond to the grayed-out lined lateral zones 461 in the lateral image of figure 33 (the bone localization profiles 450 and 460 are shown as dashed lines). Here no frontal zone can be seen.

Fig. 33 shows an example of lateral bone localization profile along patient profile of interest corresponding to figure 29. Vertical scanning speed is 45mm per second.

Curve 460 shows the lateral bone localization along the distance d from top of patient (summit of head) and along the thickness th of the patient.

The grayed areas correspond to profile points that reach the system lower and/or upper limits, and they correspond to the grayed-out lined lateral zones 461 in the lateral image of figure 33 (the bone localization profiles 450 and 460 are shown as dashed lines). Here, there is a unique and relatively narrow lateral zone 461.

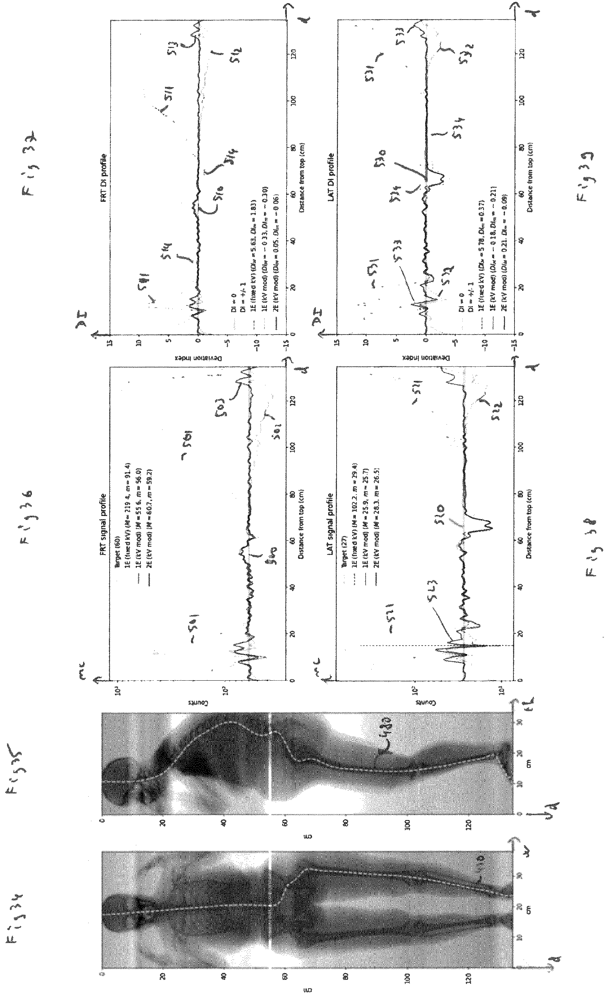

Fig. 34 shows an example of frontal bone localization profile along patient profile of interest, in a modulated image obtained through simulation. Frontal bone localization profile 470 along patient height d and along patient width w.

Fig. 35 shows an example of lateral bone localization profile along patient profile of interest, in a modulated image obtained through simulation. Lateral bone localization profile 480 along patient height d and along patient thickness th.

Fig. 36 shows an example of simulation of frontal signal profile along patient profile of interest. Frontal signal profile expressed in number of counts nc along patient height d is shown by following curves, M being the mean value and m being the median value:

- target 500 to reach,
- curve 501 for the mono-energy scout view, with a current modulation and without voltage modulation, which gives the worst result,
- curve 502 for the mono-energy scout view, with a current modulation and with a voltage modulation, which gives an intermediate result,
- curve 503 for the multi-energy (here dual-energy) scout view, with a current modulation and with a voltage modulation, which gives the best result.

Fig. 37 shows an example of simulation of frontal deviation index profile along patient profile of interest.

Frontal deviation index profile expressed in deviation index DI along patient height d is shown by following curves, M being the mean value and m being the median value:

- target 510 to reach,
- curves 514 corresponding respectively to deviations of + or - 1 from the target 500,
- curve 511 for the mono-energy scout view, with a current modulation and without voltage modulation, which gives the worst result,
- curve 512 for the mono-energy scout view, with a current modulation and with a voltage modulation, which gives an intermediate result,
- curve 513 for the multi-energy (here dual-energy) scout view, with a current modulation and with a voltage modulation, which gives the best result.

Fig. 38 shows an example of simulation of lateral signal profile along patient profile of interest.

Lateral signal profile expressed in number of counts nc along patient height d (starting from the top of patient) is shown by following curves, M being the mean value and m being the median value:

- target 520 to reach,
- curve 521 for the mono-energy scout view, with a current modulation and without voltage modulation, which gives the worst result,
- curve 522 for the mono-energy scout view, with a current modulation and with a voltage modulation, which gives an intermediate result,

- curve 523 for the multi-energy (here dual-energy) scout view, with a current modulation and with a voltage modulation, which gives the best result.

Fig. 39 shows an example of simulation of lateral deviation index profile along patient profile of interest.

Lateral deviation index profile expressed in deviation index DI along patient height d (starting from the top of patient) is shown by following curves, M being the mean value and m being the median value:

- target 530 to reach,
- curves 534 corresponding respectively to deviations of + or - 1 from the target 530,
- curve 531 for the mono-energy scout view, with a current modulation and without voltage modulation, which gives the worst result,
- curve 532 for the mono-energy scout view, with a current modulation and with a voltage modulation, which gives an intermediate result,
- curve 533 for the multi-energy (here dual-energy) scout view, with a current modulation and with a voltage modulation, which gives the best result.

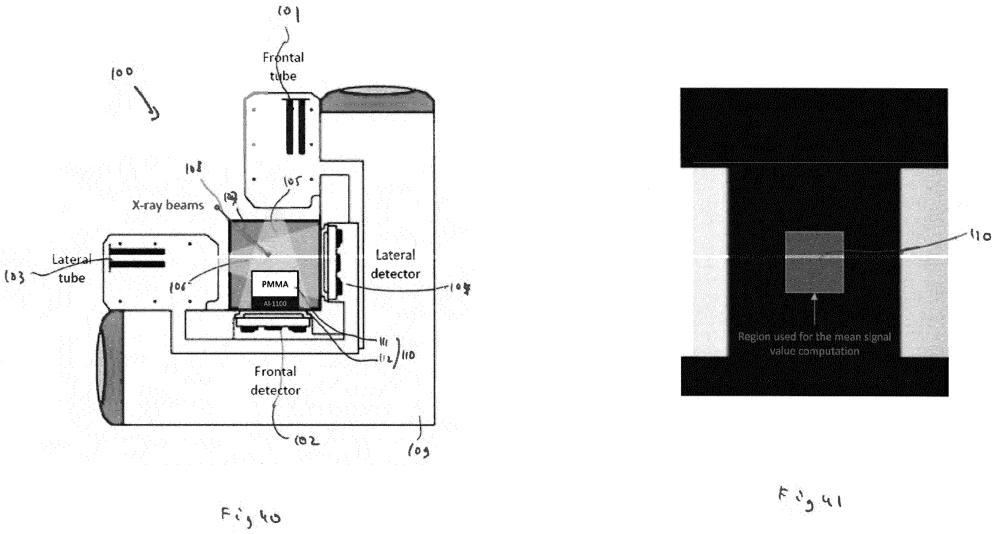

Figures 40 to 45 will now show experimentation results for the radiological imaging method according to an embodiment of the invention.

Fig. 40 shows an example of structure of imaging device to implement the radiological imaging method according to an embodiment of the invention. Vertical scanning direction is orthogonal to plan of figure 40. Patient height is also orthogonal to plan of figure 40. Vertical scanning direction is the patient height scanning direction, so a scanning direction along patient height with a standing patient.

There is a phantom 110, including a PMMA part 111 and an Al part 112, which is located on an imaging zone 107 of an imaging apparatus or an imaging device 109.

A frontal emission and reception line includes a frontal tube 101 which emits an X-ray frontal beam 105 which goes through phantom 110 and arrives on frontal detector 102.

A lateral emission and reception line includes a lateral tube 103 which emits an X-ray lateral beam 106 which goes through phantom 110 and arrives on lateral detector 104.

To avoid cross scattering in intersection zone 108, mechanical correction with vertical gap between frontal and lateral emission and reception lines and/or software cross-scattering correction between frontal and lateral emission and reception lines can be used, as mentioned earlier.

Fig. 41 shows an example of region of detector used for mean value computation during implementation of the radiological imaging method according to an embodiment of the invention. The zone 120 is the region used for the mean signal value computation.

Process to get at experimentation results is as follows:

- combine a known PMMA thickness with a known Al-1100 thickness,
- get the equivalent PMMA thickness from the mono-energy scout view,
- get a mono-energy image using the values of voltage, current, and vertical scan speed, given by the equivalent PMMA thickness (for the mono-energy scout view),
  In this case, the signal-to-thickness mapping maps total-energy scout views to PMMA thicknesses, and this mapping is then used online,
- get a mono-energy image using the values of voltage, current, and vertical scan speed, given by the (PMMA, Al) thicknesses pair (for the dual-energy scout view),
- measure the mean signal value in the central part of the image, denoted M(S), as shown on figure 41,
- compute the deviation index between this value and the target signal value; the deviation index is defined as:

$$DI_M = 10 \log_{10}\left(\frac{M(S)}{S_T}\right)$$

where $S_T$ is the target detector signal value; the closer to zero the deviation index, the better the exposure quality.

Experimental results shows the high interest of dual-energy scout view by showing that:

- the further the setup from the mono-energy scout view based model (predominant PMMA), the further the deviation index from zero,

- whereas the knowledge of (PMMA, Al) thicknesses from the dual-energy scout view based model stabilizes the deviation index around zero whatever the tested setup.

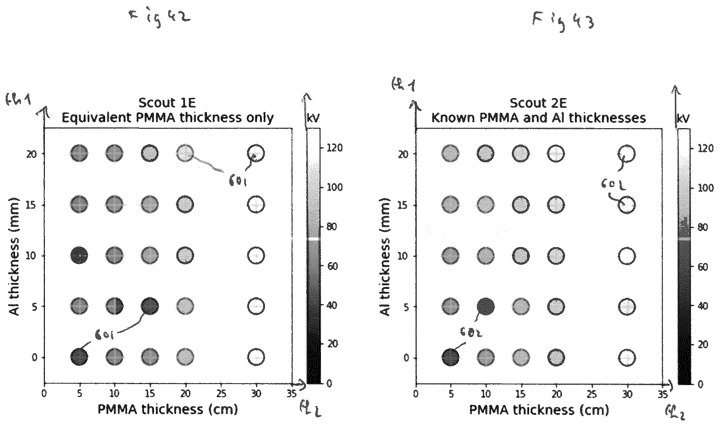

Fig. 42 shows an example of optimal kV mapping during the radiological imaging method not according to an embodiment of the invention, the scout view being mono-energy.

For each setup, an optimal voltage (kV) value is computed from the mono-energy-based equivalent PMMA thickness vectors, using the CNRD-based kV map. The gray scale of the spots 601 in the grid corresponds to the values of the voltage on the right-sided vertical voltage bar. Various couples of Al thickness th1 and PMMA thickness th2 are contemplated. Lower voltages are used for lower PMMA thicknesses and higher voltages are used higher PMMA thicknesses.

Fig. 43 shows an example of optimal kV mapping during the radiological imaging method according to an embodiment of the invention, the scout view being multi-energy.

For each setup, an optimal voltage (kV) value is computed from the dual-energy-based (PMMA, Al) thickness vector pairs, using the CNRD-based kV map. The gray scale of the spots 602 in the grid corresponds to the values of the voltage on the right-sided vertical voltage bar. Various couples of Al thickness th1 and PMMA thickness th2 are contemplated. Lower voltages are used for lower PMMA thicknesses and also somewhat for lower Al thicknesses and higher voltages are used higher PMMA thicknesses and also somewhat for higher Al thicknesses.

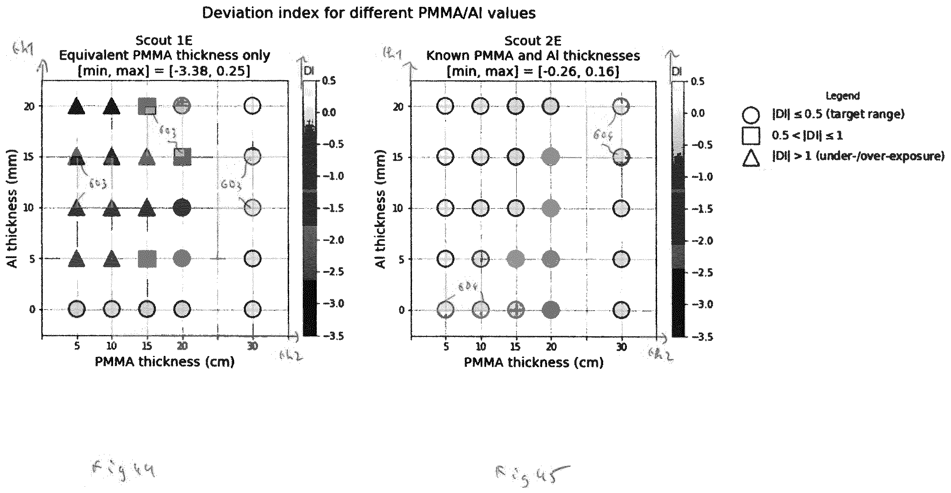

Fig. 44 shows an example of deviation index map for an optimal kV mapping during the radiological imaging method not according to an embodiment of the invention, the scout view being mono-energy.

A small deviation index is represented by a circle, an intermediate deviation index is represented by a square, a big deviation index is represented by a triangle. The gray scale of the spots 603 in the grid corresponds to the values of the deviation index on the right-sided vertical deviation index bar. Various couples of Al thickness th1 and PMMA thickness th2 are contemplated. Lower deviation index values are obtained for higher PMMA thicknesses and also for lower Al thicknesses and higher deviation index values are obtained for lower PMMA thicknesses and also for higher Al thicknesses. Some spots 603 are circles but most of them are square or triangles, what is not optimal. With only the knowledge of an equivalent PMMA thickness from mono-energy scout views, the deviation index goes awry whenever the contribution of Al becomes predominant with respect to PMMA (squares and triangles).

The non-monotonic results at high Al thickness around 15-20cm PMMA can be explained by the variable selection of the kV value.

Fig. 45 shows an example of deviation index map for an optimal kV mapping during the radiological imaging method according to an embodiment of the invention, the scout view being multi-energy.

A small deviation index is represented by a circle, an intermediate deviation index is represented by a square, a big deviation index is represented by a triangle. The gray scale of the spots 604 in the grid corresponds to the values of the deviation index on the right-sided vertical deviation index bar. Various couples of Al thickness th1 and PMMA thickness th2 are contemplated. Small and therefore good deviation index values are obtained both for high and low PMMA thicknesses and also both for high and low Al thicknesses. All spots 604 are circles, what is optimal. On figure 45, the knowledge of (Al, PMMA) thicknesses from dual-energy scout views provides exposure parameters that lead to deviation indices that are much closer to zero that for figure 44, and therefore much better.

The invention has been described with reference to preferred embodiments. However, many variations are possible within the scope of the invention.

**Claims**

1.  Radiological imaging method comprising:

    ➢ 2 radiation sources (101, 103) with imaging directions orthogonal to each other, one frontal radiation source (101) and one lateral radiation source (103), sliding vertically so as to perform vertical scanning of a standing patient along a vertical scanning direction,
    ➢ 2 radiation detectors (102, 104) which are respectively associated with said 2 radiations sources (101, 103), one frontal radiation detector (102) and one lateral radiation detector (104), sliding vertically so as to perform vertical scanning of a standing patient along said vertical scanning direction, at least said frontal radiation detector (102, 104) being a multi-energy counting detector,

    wherein said radiological method comprises at least one operating mode in which:

    ➢ a frontal multi-energy scout view is made (1) by performing a preliminary vertical scanning of a standing patient along said vertical scanning direction by said frontal radiation source (101) and by said frontal radiation detector (102), so that said frontal radiation detector (102) gives at least:

   ◦ a first frontal scout view corresponding to a first portion of energy which is received by said frontal radiation detector (102) and which is below a first given energy threshold, called low energy frontal scout view,
   ◦ a second frontal scout view corresponding to a second portion of energy which is received by said frontal radiation detector (102) and which is above a second given energy threshold, called high energy frontal scout view,

➢ said first frontal scout view and said second frontal scout view are combined and processed (20) so as to evaluate:

   ◦ at least a patient bone thickness (22),
   ◦ at least a patient soft tissue thickness (22),
   ◦ a patient specific bone localization at different imaging positions along said vertical scanning direction (21),

➢ a frontal image is made (3) by performing a vertical scanning of a standing patient along said vertical scanning direction by said frontal radiation source (101) and by said frontal radiation detector (102), with:

   ◦ a modulation of a driving current intensity of at least said frontal radiation source (101) along said vertical scanning direction, depending on said patient bone thickness, on said patient soft tissue thickness, and on said patient specific bone localization at different imaging positions along said vertical scanning direction,
   ◦ and preferably also a modulation of a driving voltage intensity of said frontal radiation source (101) along said vertical scanning direction, depending on said patient bone thickness, on said patient soft tissue thickness, and on said patient specific bone localization at different imaging positions along said vertical scanning direction,
   ◦ either driving current intensity modulation of said frontal radiation source (101), with no voltage intensity modulation of said frontal radiation source (101), is performed automatically, so as to improve a compromise between:

      ■ the global radiation dose received by a patient during said vertical scanning,
      ■ and the local image contrasts of said identified specific bone(s) localization at different imaging positions along said vertical scanning direction, for the frontal image,

   ◦ or both driving current intensity and voltage intensity modulations of said frontal radiation source (101) are performed simultaneously, preferably synchronously, and automatically, so as to improve a compromise between:

      ■ the global radiation dose received by a patient during said vertical scanning,
      ■ and the local image contrasts of said identified specific bone(s) localization at different imaging positions along said vertical scanning direction, for the frontal image.

2.  Radiological imaging method comprising:

➢ 2 radiation sources (101, 103) with imaging directions orthogonal to each other, one frontal radiation source (101) and one lateral radiation source (103), sliding vertically so as to perform vertical scanning of a standing patient along a vertical scanning direction,
➢ 2 radiation detectors (102, 104) which are respectively associated with said 2 radiations sources (101, 103), one frontal radiation detector (102) and one lateral radiation detector (104), sliding vertically so as to perform vertical scanning of a standing patient along said vertical scanning direction, at least said lateral radiation detector (104) being a multi-energy counting detector,

wherein said radiological method comprises at least one operating mode in which:

➢ a lateral multi-energy scout view is made (1) by performing a preliminary vertical scanning of a standing patient along said vertical scanning direction by said lateral radiation source (103) and by said lateral radiation detector (104), so that said lateral radiation detector (104) gives at least:

   ◦ a first lateral scout view corresponding to a first portion of energy which is received by said lateral radiation detector (104) and which is below a first given energy threshold, called low energy lateral scout view,

◦ a second lateral scout view corresponding to a second portion of energy which is received by said lateral radiation detector (104) and which is above a second given energy threshold, called high energy lateral scout view,

➣ said first lateral scout view and said second lateral scout view are combined and processed (20) so as to evaluate:

◦ at least a patient bone thickness (22),
◦ at least a patient soft tissue thickness (22),
◦ a patient specific bone localization at different imaging positions along said vertical scanning direction (21),

➣ a lateral image is made (3) by performing a vertical scanning of a standing patient along said vertical scanning direction by said lateral radiation source (103) and by said lateral radiation detector (104), with:

◦ a modulation of a driving current intensity of at least said lateral radiation source (103) along said vertical scanning direction, depending on said patient bone thickness, on said patient soft tissue thickness, and on said patient specific bone localization at different imaging positions along said vertical scanning direction,
◦ and preferably also a modulation of a driving voltage intensity of said lateral radiation source (103) along said vertical scanning direction, depending on said patient bone thickness, on said patient soft tissue thickness, and on said patient specific bone localization at different imaging positions along said vertical scanning direction,
◦ either driving current intensity modulation of said lateral radiation source (103), with no voltage intensity modulation of said lateral radiation source (103), is performed automatically, so as to improve a compromise between:

▪ the global radiation dose received by a patient during said vertical scanning,
▪ and the local image contrasts of said identified specific bone(s) localization at different imaging positions along said vertical scanning direction, for the lateral image,

◦ or both driving current intensity and voltage intensity modulations of said lateral radiation source (103) are performed simultaneously, preferably synchronously, and automatically, so as to improve a compromise between:

▪ the global radiation dose received by a patient during said vertical scanning,
▪ and the local image contrasts of said identified specific bone(s) localization at different imaging positions along said vertical scanning direction, for the lateral image.

3. Radiological imaging method comprising:

➣ 2 radiation sources (101, 103) with imaging directions orthogonal to each other, one frontal radiation source (101) and one lateral radiation source (103), sliding vertically so as to perform vertical scanning of a standing patient along a vertical scanning direction,
➣ 2 radiation detectors (102, 104) which are respectively associated with said 2 radiations sources (101, 103), one frontal radiation detector (102) and one lateral radiation detector (104), sliding vertically so as to perform vertical scanning of a standing patient along said vertical scanning direction, said 2 radiation detectors (102, 104) being respectively 2 multi-energy counting detectors,

wherein said radiological method comprises at least one operating mode in which:

➣ frontal and lateral multi-energy scout views are made (1) by performing a preliminary vertical scanning of a standing patient along said vertical scanning direction by said frontal and lateral radiation sources (101, 103) and by said frontal and lateral radiation detectors (102, 104), so that said frontal and lateral radiation detectors (102, 104) give at least:

◦ a first frontal scout view corresponding to a first portion of energy which is received by said frontal radiation detector (102) and which is below a first given energy threshold, called low energy frontal scout view,
◦ a second frontal scout view corresponding to a second portion of energy which is received by said frontal radiation detector (102) and which is above a second given energy threshold, called high energy frontal scout view,

◦ a first lateral scout view corresponding to a first portion of energy which is received by said lateral radiation detector (104) and which is below a first given energy threshold, called low energy lateral scout view,

◦ a second lateral scout view corresponding to a second portion of energy which is received by said lateral radiation detector (104) and which is above a second given energy threshold, called high energy lateral scout view,

➢ said first frontal and lateral scout views and said second frontal and lateral scout views are combined and processed (20) so as to evaluate:

◦ at least a patient bone thickness (22),
◦ at least a patient soft tissue thickness (22),
◦ a patient specific bone localization at different imaging positions along said vertical scanning direction (21),

➢ a frontal image is made (3) by performing a vertical scanning of a standing patient along said vertical scanning direction by said frontal radiation source (101) and by said frontal radiation detector (102), and a lateral image is made by performing a vertical scanning of a standing patient along said vertical scanning direction by said lateral radiation source (103) and by said lateral radiation detector (104), both frontal and lateral images being made during same vertical scanning, with:

◦ a modulation of driving current intensities of both said frontal and lateral radiation sources (101, 103) along said vertical scanning direction, depending on said patient bone thickness, on said patient soft tissue thickness, and on said patient specific bone localization at different imaging positions along said vertical scanning direction,

◦ and preferably also a modulation of driving voltage intensities of both said frontal and lateral radiation sources (101, 103) along said vertical scanning direction, depending on said patient bone thickness, on said patient soft tissue thickness, and on said patient specific bone localization at different imaging positions along said vertical scanning direction,

◦ either driving current intensity modulation of said frontal radiation source (101), with no voltage intensity modulation of said frontal radiation source (101), as well as driving current intensity modulation of said lateral radiation source (103), with no voltage intensity modulation of said lateral radiation source (103), are performed simultaneously, preferably synchronously, and automatically, so as to improve a compromise between:

▪ the global radiation dose received by a patient during said vertical scanning,
▪ and the local image contrasts of said identified specific bone(s) localization at different imaging positions along said vertical scanning direction, for the frontal image and for the lateral image,

◦ or both driving current intensity and voltage intensity modulations of said frontal radiation source (101), are performed simultaneously, preferably synchronously, and automatically, as well as both driving current intensity and voltage intensity modulations of said lateral radiation source (103), are performed simultaneously, preferably synchronously, and automatically, so as to improve a compromise between:

▪ the global radiation dose received by a patient during said vertical scanning,
▪ and the local image contrasts of said identified specific bone(s) localization at different imaging positions along said vertical scanning direction, for the frontal image and for the lateral image.

4. Radiological imaging method according to any of preceding claims, wherein:

➢ said frontal multi-energy scout view is made (1) by performing a single preliminary vertical scanning of a standing patient along said vertical scanning direction by said frontal radiation source (101) and by said frontal radiation detector (102), so that said frontal radiation detector (102) gives at least:

◦ said first frontal scout view,
◦ said second frontal scout view.

➢ said lateral multi-energy scout view is made (1) by performing a single preliminary vertical scanning of a standing patient along said vertical scanning direction by said lateral radiation source (103) and by said lateral radiation detector (104), so that said lateral radiation detector (104) gives at least:

  ◦ said first lateral scout view,
  ◦ said second lateral scout view.

➢ both said frontal multi-energy scout view and said lateral multi-energy scout view being made during same single preliminary vertical scanning.

5. Radiological imaging method according to any of preceding claims, wherein:

➢ said frontal image is made (3) by performing a single vertical scanning of a standing patient along said vertical scanning direction by said frontal radiation source (101) and by said frontal radiation detector (102),
➢ said lateral image is made (3) by performing a single vertical scanning of a standing patient along said vertical scanning direction by said lateral radiation source (103) and by said lateral radiation detector (104),
➢ both said frontal image and said lateral image being made during same single vertical scanning.

6. Radiological imaging method according to any of preceding claims, wherein said first given energy threshold is equal to or less than said second given energy threshold, preferably equal to said second given energy threshold, and preferably wherein:

➢ said first given energy threshold is equal to said second given energy threshold,
➢ said frontal and/or lateral multi-energy scout views are made (1) so that said frontal and/or lateral radiation detectors (102, 104) first give:

  ◦ said first frontal scout view,
  ◦ a third frontal scout view corresponding to the whole energy which is received by said frontal radiation detector (102), called total energy frontal scout view,

    ▪ said second frontal scout view being obtained by a subtracting said first frontal scout view from said third frontal scout view,

  ◦ and/or said first lateral scout view,
  ◦ and/or a third lateral scout view corresponding to the whole energy which is received by said lateral radiation detector (104), called total energy lateral scout view,

    ▪ said second lateral scout view being obtained by a subtracting said first lateral scout view from said third lateral scout view.

7. Radiological imaging method according to any of preceding claims, wherein:

➢ said first frontal and/or lateral scout view(s) and said second frontal and/or lateral scout view(s) are combined and processed so as to evaluate (22) a patient bone thickness profile along said vertical scanning direction,
➢ and/or said first frontal and/or lateral scout view(s) and said second frontal and/or lateral scout view(s) are combined and processed so as to evaluate (22) a patient soft tissue thickness profile along said vertical scanning direction,
➢ said frontal image is made by performing a vertical scanning of a standing patient along said vertical scanning direction by said frontal radiation source (101) and by said frontal radiation detector (102), with:

  ◦ a modulation of a driving current intensity of at least said frontal radiation source (101) along said vertical scanning direction, depending on said patient bone thickness profile and/or on said patient soft tissue thickness profile along said vertical scanning direction,
  ◦ and preferably also said modulation of a driving voltage intensity of said frontal radiation source (101) along said vertical scanning direction, depending on said patient bone thickness profile and on said patient soft tissue thickness profile along said vertical scanning direction,

➢ said lateral image is made by performing a vertical scanning of a standing patient along said vertical scanning direction by said lateral radiation source (103) and by said lateral radiation detector (104), with:

◦ a modulation of a driving current intensity of at least said lateral radiation source (103) along said vertical scanning direction, depending on said patient bone thickness profile and/or on said patient soft tissue thickness profile along said vertical scanning direction,

◦ and preferably also said modulation of a driving voltage intensity of said lateral radiation source (103) along said vertical scanning direction, depending on said patient bone thickness profile and on said patient soft tissue thickness profile along said vertical scanning direction,

➢ both said frontal image and said lateral image being made during same vertical scanning.

8. Radiological imaging method according to any of preceding claims, wherein:

◦ either said driving current intensity modulation(s) of said frontal and/or lateral radiation source(s) (101, 103), with no voltage intensity modulation of said frontal and/or lateral radiation source(s) (101, 103), is performed automatically, so as to improve a compromise between:

▪ lowering the global radiation dose received by a patient during said vertical scanning,
▪ and not degrading under a given contrast threshold the local image contrasts of said identified specific bone(s) localization at different imaging positions along said vertical scanning direction, for all or part of patient thicknesses along said vertical scanning direction, for the frontal and/or lateral image(s),

◦ or said both driving current intensity and voltage intensity modulations of said frontal and/or lateral radiation source(s) (101, 103) are performed simultaneously, preferably synchronously, and automatically, so as to improve a compromise between:

▪ lowering the global radiation dose received by a patient during said vertical scanning,
▪ and increasing, the local image contrasts of said identified specific bone(s) localization at different imaging positions along said vertical scanning direction, with respect to local image contrasts of said identified specific bone(s) localization at different imaging positions along said vertical scanning direction with same global radiation dose but without any driving current intensity modulation nor any driving voltage intensity modulation, for all or part of patient thicknesses along said vertical scanning direction, for the frontal and/or lateral image(s),

and/or wherein:

◦ either said driving current intensity modulation(s) of said frontal and/or lateral radiation source(s) (101, 103), with no voltage intensity modulation of said frontal and/or lateral radiation source(s) (101, 103), is performed automatically, so as to improve a compromise between:

▪ lowering the global radiation dose received by a patient during said vertical scanning,
▪ and improving the contrast to noise ratio or the ratio between contrast to noise ratio and square root of said global radiation dose of said identified specific bone(s) localization at different imaging positions along said vertical scanning direction, with respect to local image contrasts of said identified specific bone(s) localization at different imaging positions along said vertical scanning direction with same global radiation dose but without any driving current intensity modulation, for all or part of patient thicknesses along said vertical scanning direction, for the frontal and/or lateral image(s),

◦ or said both driving current intensity and voltage intensity modulations of said frontal and/or lateral radiation source(s) (101, 103) are performed simultaneously, preferably synchronously, and automatically, so as to improve a compromise between:

▪ lowering the global radiation dose received by a patient during said vertical scanning,
▪ and improving the contrast to noise ratio or the ratio between contrast to noise ratio and square root of said global radiation dose of said identified specific bone(s) localization at different imaging positions along said vertical scanning direction, with respect to local image contrasts of said identified specific bone(s) localization at different imaging positions along said vertical scanning direction with same global radiation dose but without any driving current intensity modulation nor any driving voltage intensity modulation, for all or part of patient thicknesses along said vertical scanning direction, for the frontal and/or lateral image(s).

9. Radiological imaging method according to any of preceding claims, wherein:

> said frontal multi-energy scout view acquisition is performed (1) with at least 2 energy bins, or with at least 3 energy bins, or with at least 6 energy bins,

○ and/or at most 20 energy bins, or at most 15 energy bins, or at most 10 energy bins,

> and/or said lateral multi-energy scout view acquisition is performed (1) with at least 2 energy bins, or with at least 3 energy bins, or with at least 6 energy bins,

○ and/or at most 20 energy bins, or at most 15 energy bins, or at most 10 energy bins.

10. Radiological imaging method according to any of preceding claims, wherein:

> said first and second frontal scout views are processed to a multi-material decomposition with at least two material thickness vertical profiles,

○ preferably, either a bi-material decomposition between Al and PMMA, or a bi-material decomposition between HA and $H_2O$,

> and/or said first and second lateral scout views are processed to a multi-material decomposition with at least two material thickness vertical vectors,

○ preferably, either a bi-material decomposition between Al and PMMA, or a bi-material decomposition between HA and $H_2O$.

11. Radiological imaging method according to any of preceding claims, wherein said both driving current intensity and voltage intensity modulations of said frontal and/or lateral radiation source(s) (101, 103) are performed also so as to reach a value of signal to noise ratio which is constant and common to most of said imaging positions along said vertical scanning direction, preferably to all said imaging positions along said vertical scanning direction, for said frontal image and/or for said lateral image, but which can take two different values respectively for frontal image and for lateral image.

12. Radiological imaging method according to claim 11, wherein, for each of said frontal and/or lateral images, said signal to noise ratio value is constant and predetermined for each different patient organ to be imaged, and/or wherein:

> for a frontal image of a patient spine, said standard signal to noise ratio value corresponds to a number of X-ray photons received per detector pixel comprised between 50 and 70, the radiological imaging method operator preferably having the possibility to deviate, via a manual command, from this standard value by at least -25% or +100%, more preferably by at least -50% or +200%,
> and/or for a lateral image of a patient spine, said standard signal to noise ratio value corresponds to a number of X-ray photons received per detector pixel comprised between 20 and 40, the radiological imaging method operator preferably having the possibility to deviate, via a manual command, from this standard value by at least -25% or +100%, more preferably by at least -50% or +200%.

13. Radiological imaging method according to any of preceding claims, wherein:

> modulations of both current intensity and voltage intensity:

○ simultaneously increase both current intensity and voltage intensity for bigger patient thicknesses,
○ simultaneously decrease both current intensity and voltage intensity for smaller patient thicknesses,
○ current intensity variation rate being slower than voltage intensity variation rate.

14. Radiological imaging method according to any of preceding claims, wherein said current intensity modulation is maximized so as to also maximize said vertical scanning speed at a constant value.

15. Radiological imaging method according to any of preceding claims, wherein said current intensity modulation(s) rate do(es) not go beyond a predetermined threshold of 5 mA per millisecond, or do(es) not go beyond a predetermined threshold of 2 mA per millisecond, or do(es) not go beyond a predetermined threshold of 1 mA per millisecond,

and/or wherein said current intensity modulation(s) at least range(s) from 20mA to 300mA, and preferably from 10mA to 400mA,
and/or wherein said voltage intensity modulation(s) at least range(s) from 60kV to 100kV, and preferably from 50kV to 130kV,
and/or wherein said vertical scanning speed value at least range(s) from 8cm/second to 20cm/second, and preferably from 0.4cm/second to 35cm/second.

16. Radiological imaging method according to any of preceding claims, wherein each of said frontal and/or lateral scout view(s) is made (1) by performing a preliminary vertical scanning of a standing patient along a vertical scanning direction with a reduced global radiation dose as compared to each of said frontal and lateral images, before making each of said frontal and lateral images, and preferably wherein said reduced global radiation is less than 10% of said global radiation dose, more preferably less than 5% of said global radiation dose.

17. Radiological imaging method according to any of preceding claims, wherein pixels in said scout view are gathered together, preferably by zones of NxN pixels, more preferably by zones ranging from 2x2 pixels to of 10x10 pixels, to make imaged zones.

18. Radiological imaging method according to any of preceding claims, wherein said images or said imaged zones are processed to identify salient points (60) which in turn are used to compute (22) said thickness profile(s) and to identify (21) said specific bone(s) localization of a standing patient along said vertical scanning direction.

19. Radiological imaging method according to any of preceding claims, wherein said 2 radiation detectors (102, 104) are respectively associated with said 2 radiations sources (101, 103), said 2 radiation detectors (102, 104) being 2 Photon Counting Detectors (PCD) each being associated to an automatic image processing function automatically balancing image gray levels whatever radiation dose received on the sensitive surface of said radiation detector (102, 104) to homogenize responses of said detectors (102, 104),
and/or wherein said 2 radiation detectors (102, 104) are respectively associated with said 2 radiations sources (101, 103), said 2 radiation detectors (102, 104) being 2 multi-energy counting detectors, preferably being 2 Energy Resolved Photon Counting Detectors (ERPCD).

20. Radiological imaging method according to any of preceding claims, wherein said second energy threshold is chosen so as to improve image contrast more for lower patient thicknesses regions along vertical direction than for higher patient thicknesses regions along vertical direction, preferably said second energy threshold being chosen between 50keV and 90keV, preferably between 60keV and 80keV, more preferably said second energy threshold being chosen at 70keV.

21. Radiological imaging method according to any of preceding claims, wherein:

➢ said frontal image and/or said lateral image are both mono-energy images, performed (3) with said voltage intensity modulation(s) of said frontal radiation source (101) and/or of said lateral radiation source (103),
➢ or said frontal image and/or said lateral image are both multi-energy images, performed with no voltage intensity modulation, of said frontal radiation source (101) and/or of said lateral radiation source (103).

**Patentansprüche**

1. Radiologisches Bildgebungsverfahren, umfassend:

- zwei Strahlungsquellen (101, 103) mit Bildgebungsrichtungen, welche orthogonal zueinander sind, eine frontale Strahlungsquelle (101) und eine laterale Strahlungsquelle (103), welche vertikal gleiten, um eine vertikale Abtastung eines stehenden Patienten entlang einer vertikalen Abtastrichtung durchzuführen;
- zwei Strahlungsdetektoren (102, 104), welche jeweils mit den zwei Strahlungsquellen (101, 103) verbunden sind, ein frontaler Strahlungsdetektor (102) und ein lateraler Strahlungsdetektor (104), welche vertikal gleiten, um eine vertikale Abtastung eines stehenden Patienten entlang der vertikalen Abtastrichtung durchzuführen, wobei wenigstens der frontale Strahlungsdetektor (102, 104) ein Multienergie-Zähldetektor ist;

wobei das radiologische Verfahren wenigstens einen Betriebsmodus umfasst, in welchem:

- eine frontale Multienergie-Vorabansicht (1) durch Durchführen einer vorläufigen vertikalen Abtastung eines stehenden Patienten entlang der vertikalen Abtastrichtung durch die frontale Strahlungsquelle (101) und durch den frontalen Strahlungsdetektor (102) erzeugt wird, sodass der frontale Strahlungsdetektor (102) wenigstens ausgibt:

eine erste frontale Vorabansicht, welche einem ersten Energieanteil entspricht, welcher von dem frontalen Strahlungsdetektor (102) empfangen wird und welcher unterhalb eines ersten vorgegebenen Energieschwellenwerts liegt, welcher als niederenergetische frontale Vorabansicht bezeichnet wird;
eine zweite frontale Vorabansicht, welche einem zweiten Energieanteil entspricht, welcher von dem frontalen Strahlungsdetektor (102) empfangen wird und welcher oberhalb eines zweiten vorgegebenen Energieschwellenwerts liegt, welcher als hochenergetische frontale Vorabansicht bezeichnet wird;

- die erste frontale Vorabansicht und die zweite frontale Vorabansicht kombiniert und verarbeitet werden (20), um abzuschätzen:

wenigstens eine Patientenknochen-Dicke (22),
wenigstens eine Patient-Weichgewebe-Dicke (22),
eine patientenspezifische Knochenlokalisation an verschiedenen Bildgebungspositionen entlang der vertikalen Abtastrichtung (21);

- ein frontales Bild (3) durch Durchführen einer vertikalen Abtastung eines stehenden Patienten entlang der vertikalen Abtastrichtung durch die frontale Strahlungsquelle (101) und durch den frontalen Strahlungsdetektor (102) erzeugt wird, mit:

◦ einer Modulation einer Ansteuerstromstärke von wenigstens der frontalen Strahlungsquelle (101) entlang der vertikalen Abtastrichtung, abhängig von der Patientenknochen-Dicke, der Patient-Weichgewebe-Dicke und der patientenspezifischen Knochenlokalisation an verschiedenen Bildgebungspositionen entlang der vertikalen Abtastrichtung;
◦ und vorzugsweise auch einer Modulation einer Ansteuerspannungsstärke der frontalen Strahlungsquelle (101) entlang der vertikalen Abtastrichtung, abhängig von der Patientenknochen-Dicke, der Patient-Weichgewebe-Dicke und der patientenspezifischen Knochenlokalisation an verschiedenen Bildgebungspositionen entlang der vertikalen Abtastrichtung;
◦ wobei entweder eine Ansteuerstromstärkenmodulation der frontalen Strahlungsquelle (101), ohne Ansteuerspannungsstärkenmodulation der frontalen Strahlungsquelle (101), automatisch durchgeführt wird, um einen Kompromiss zu verbessern zwischen:

▪ der globalen Strahlendosis, welche ein Patient während der vertikalen Abtastung empfängt, und
▪ den lokalen Bildkontrasten der identifizierten spezifischen Knochenlokalisation(en) an verschiedenen Bildgebungspositionen entlang der vertikalen Abtastrichtung, für das frontale Bild;

◦ oder die Modulation der Ansteuerstromstärke und der Ansteuerspannungsstärke der frontalen Strahlungsquelle (101) simultan, vorzugsweise synchron, und automatisch, durchgeführt werden, um einen Kompromiss zu verbessern zwischen:

▪ der globalen Strahlendosis, welche ein Patient während der vertikalen Abtastung empfängt, und
▪ den lokalen Bildkontrasten der identifizierten spezifischen Knochenlokalisation an verschiedenen Bildgebungspositionen entlang der vertikalen Abtastrichtung, für das frontale Bild.

2. Radiologisches Bildgebungsverfahren, umfassend:

- zwei Strahlungsquellen (101, 103) mit Bildgebungsrichtungen, welche orthogonal zueinander sind, eine frontale Strahlungsquelle (101) und eine laterale Strahlungsquelle (103), welche vertikal gleiten, um eine vertikale Abtastung eines stehenden Patienten entlang einer vertikalen Abtastrichtung durchzuführen;
- zwei Strahlungsdetektoren (102, 104), welche jeweils mit den zwei Strahlungsquellen (101, 103) verbunden sind, ein frontaler Strahlungsdetektor (102) und ein lateraler Strahlungsdetektor (104), welche vertikal gleiten, um eine vertikale Abtastung eines stehenden Patienten entlang der vertikalen Abtastrichtung durchzuführen, wobei wenigstens der laterale Strahlungsdetektor (104) ein Multienergie-Zähldetektor ist;

wobei das radiologische Verfahren wenigstens einen Betriebsmodus umfasst, in welchem:

- eine laterale Multienergie-Vorabansicht (1) durch Durchführen einer vorläufigen vertikalen Abtastung eines stehenden Patienten entlang der vertikalen Abtastrichtung durch die laterale Strahlungsquelle (103) und durch den lateralen Strahlungsdetektor (104) erzeugt wird, sodass der laterale Strahlungsdetektor (104) wenigstens ausgibt:

◦ eine erste laterale Vorabansicht, welche einem ersten Energieanteil entspricht, welcher von dem lateralen Strahlungsdetektor (104) empfangen wird und unterhalb eines ersten vorgegebenen Energieschwellenwerts liegt, welcher als niederenergetische laterale Vorabansicht bezeichnet wird;
◦ eine zweite laterale Vorabansicht, welche einem zweiten Energieanteil entspricht, welcher von dem lateralen Strahlungsdetektor (104) empfangen wird und oberhalb eines zweiten vorgegebenen Energieschwellenwerts liegt, welcher als hochenergetische laterale Vorabansicht bezeichnet wird;

- die erste laterale Vorabansicht und die zweite laterale Vorabansicht kombiniert und verarbeitet werden (20), um abzuschätzen:

◦ wenigstens eine Patientenknochen-Dicke (22),
◦ wenigstens eine Patient-Weichgewebe-Dicke (22),
◦ eine patientenspezifische Knochenlokalisation an verschiedenen Bildgebungspositionen entlang der vertikalen Abtastrichtung (21);

- ein laterales Bild (3) durch Durchführen einer vertikalen Abtastung eines stehenden Patienten entlang der vertikalen Abtastrichtung durch die laterale Strahlungsquelle (103) und durch den lateralen Strahlungsdetektor (104) erzeugt wird, mit:

◦ einer Modulation einer Ansteuerstromstärke von wenigstens der lateralen Strahlungsquelle (103) entlang der vertikalen Abtastrichtung, abhängig von der Patientenknochen-Dicke, der Patient-Weichgewebe-Dicke und der patientenspezifischen Knochenlokalisation an verschiedenen Bildgebungspositionen entlang der vertikalen Abtastrichtung;
◦ und vorzugsweise auch einer Modulation einer Ansteuerspannungsstärke der lateralen Strahlungsquelle (103) entlang der vertikalen Abtastrichtung, abhängig von der Patientenknochen-Dicke, der Patient-Weichgewebe-Dicke und der patientenspezifischen Knochenlokalisation an verschiedenen Bildgebungspositionen entlang der vertikalen Abtastrichtung;
o wobei entweder eine Ansteuerstromstärkenmodulation der lateralen Strahlungsquelle (103), ohne Ansteuerspannungsstärkenmodulation der lateralen Strahlungsquelle (103), automatisch durchgeführt wird, um einen Kompromiss zu verbessern zwischen:

■ der globalen Strahlendosis, welche ein Patient während der vertikalen Abtastung empfängt, und den lokalen Bildkontrasten der identifizierten spezifischen Knochenlokalisation an verschiedenen Bildgebungspositionen entlang der vertikalen Abtastrichtung, für das laterale Bild;

◦ oder die Modulation der Ansteuerstromstärke und der Ansteuerspannungsstärke der lateralen Strahlungsquelle (103) simultan, vorzugsweise synchron, und automatisch, durchgeführt werden, um einen Kompromiss zu verbessern zwischen:

■ der globalen Strahlendosis, welche ein Patient während der vertikalen Abtastung empfängt, und den lokalen Bildkontrasten der identifizierten spezifischen Knochenlokalisation an verschiedenen Bildgebungspositionen entlang der vertikalen Abtastrichtung, für das laterale Bild.

3. Radiologisches Bildgebungsverfahren, umfassend:

- zwei Strahlungsquellen (101, 103) mit Bildgebungsrichtungen, welche orthogonal zueinander sind, eine frontale Strahlungsquelle (101) und eine laterale Strahlungsquelle (103), welche vertikal gleiten, um eine vertikale Abtastung eines stehenden Patienten entlang einer vertikalen Abtastrichtung durchzuführen;
- zwei Strahlungsdetektoren (102, 104), welche jeweils mit den zwei Strahlungsquellen (101, 103) verbunden sind, ein frontaler Strahlungsdetektor (102) und ein lateraler Strahlungsdetektor (104), welche vertikal gleiten, um eine vertikale Abtastung eines stehenden Patienten entlang der vertikalen Abtastrichtung durchzuführen, wobei die zwei Strahlungsdetektoren (102, 104) jeweils zwei Multienergie-Zähldetektoren sind;

wobei das radiologische Verfahren wenigstens einen Betriebsmodus umfasst, in welchem:

- frontale und laterale Multienergie-Vorabansichten (1) durch Durchführen einer vorläufigen vertikalen Abtastung eines stehenden Patienten entlang der vertikalen Abtastrichtung durch die frontale und die laterale Strahlungsquelle (101, 103) und durch den frontalen und den lateralen Strahlungsdetektor (102, 104) erzeugt werden, sodass der frontale und der laterale Strahlungsdetektor (102, 104) wenigstens ausgeben:

  ◦ eine erste frontale Vorabansicht, welche einem ersten Energieanteil entspricht, welcher von dem frontalen Strahlungsdetektor (102) empfangen wird und unterhalb eines ersten vorgegebenen Energieschwellenwerts liegt, welcher als niederenergetische frontale Vorabansicht bezeichnet wird;
  ◦ eine zweite frontale Vorabansicht, welche einem zweiten Energieanteil entspricht, welcher von dem frontalen Strahlungsdetektor (102) empfangen wird und oberhalb eines zweiten vorgegebenen Energieschwellenwerts liegt, welcher als hochenergetische frontale Vorabansicht bezeichnet wird;
  ◦ eine erste laterale Vorabansicht, welche einem ersten Energieanteil entspricht, welcher von dem lateralen Strahlungsdetektor (104) empfangen wird und unterhalb eines ersten vorgegebenen Energieschwellenwerts liegt, welcher als niederenergetische laterale Vorabansicht bezeichnet wird;
  ◦ eine zweite laterale Vorabansicht, welche einem zweiten Energieanteil entspricht, welcher von dem lateralen Strahlungsdetektor (104) empfangen wird und oberhalb eines zweiten vorgegebenen Energieschwellenwerts liegt, welcher als hochenergetische laterale Vorabansicht bezeichnet wird;

- die erste frontale und laterale Vorabansicht und die zweite frontale und laterale Vorabansicht kombiniert und verarbeitet werden (20), um abzuschätzen:

  ◦ wenigstens eine Patientenknochen-Dicke (22),
  ◦ wenigstens eine Patient-Weichgewebe-Dicke (22),
  ◦ eine patientenspezifische Knochenlokalisation an verschiedenen Bildgebungspositionen entlang der vertikalen Abtastrichtung (21);

- ein frontales Bild (3) durch Durchführen einer vertikalen Abtastung eines stehenden Patienten entlang der vertikalen Abtastrichtung durch die frontale Strahlungsquelle (101) und durch den frontalen Strahlungsdetektor (102) erzeugt wird und ein laterales Bild durch Durchführen einer vertikalen Abtastung eines stehenden Patienten entlang der vertikalen Abtastrichtung durch die laterale Strahlungsquelle (103) und durch den lateralen Strahlungsdetektors (104) erzeugt wird, wobei sowohl das frontale als auch das laterale Bild während derselben vertikalen Abtastung erzeugt werden, mit:

  - einer Modulation von Ansteuerstromstärken von sowohl der frontalen als auch der lateralen Strahlungsquelle (101, 103) entlang der vertikalen Abtastrichtung, abhängig von der Patientenknochen-Dicke, der Patient-Weichgewebe-Dicke und der patientenspezifischen Knochenlokalisation an verschiedenen Bildgebungspositionen entlang der vertikalen Abtastrichtung;
  - und vorzugsweise auch einer Modulation der Ansteuerspannungsstärken von sowohl der frontalen als auch der lateralen Strahlungsquelle (101, 103) entlang der vertikalen Abtastrichtung, abhängig von der Patientenknochen-Dicke, der Patient-Weichgewebe-Dicke und der patientenspezifischen Knochenlokalisation an verschiedenen Bildgebungspositionen entlang der vertikalen Abtastrichtung;
  - wobei entweder eine Ansteuerstromstärkenmodulation der frontalen Strahlungsquelle (101), ohne Spannungsstärkenmodulation der frontalen Strahlungsquelle (101), sowie eine Ansteuerstromstärkenmodulation der lateralen Strahlungsquelle (103), ohne Spannungsstärkenmodulation der lateralen Strahlungsquelle (103), simultan, vorzugsweise synchron, und automatisch, durchgeführt wird, um einen Kompromiss zu verbessern zwischen:

    ◦ der globalen Strahlendosis, welche ein Patient während der vertikalen Abtastung empfängt, und
    ◦ den lokalen Bildkontrasten der identifizierten spezifischen Knochenlokalisation an verschiedenen Bildgebungspositionen entlang der vertikalen Abtastrichtung, für das frontale Bild und das laterale Bild;

  - oder sowohl die Ansteuerstromstärkenmodulation als auch die Ansteuerspannungsstärkenmodulation der frontalen Strahlungsquelle (101) simultan, vorzugsweise synchron, und automatisch, durchgeführt werden, sowie sowohl die Ansteuerstromstärkenmodulation als auch die Spannungsstärkenmodulation der lateralen Strahlungsquelle (103) simultan, vorzugsweise synchron, und automatisch, durchgeführt werden, um einen Kompromiss zu verbessern zwischen:

- ◦ der globalen Strahlendosis, welche ein Patient während der vertikalen Abtastung empfängt, und
- ◦ den lokalen Bildkontrasten der identifizierten spezifischen Knochenlokalisation an verschiedenen Bildgebungspositionen entlang der vertikalen Abtastrichtung, für das frontale Bild und das laterale Bild.

4. Radiologisches Bildgebungsverfahren nach einem der vorhergehenden Ansprüche, wobei:

- die frontale Multienergie-Vorabansicht (1) durch Durchführen einer einzelnen vorläufigen vertikalen Abtastung eines stehenden Patienten entlang der vertikalen Abtastrichtung durch die frontale Strahlungsquelle (101) und durch den frontalen Strahlungsdetektor (102) erzeugt wird, sodass der frontale Strahlungsdetektor (102) wenigstens ausgibt:

- ◦ die erste frontale Vorabansicht;
- ◦ die zweite frontale Vorabansicht;

- die laterale Multienergie-Vorabansicht (1) durch Durchführen einer einzelnen vorläufigen vertikalen Abtastung eines stehenden Patienten entlang der vertikalen Abtastrichtung durch die laterale Strahlungsquelle (103) und durch den lateralen Strahlungsdetektor (104) erzeugt wird, sodass der laterale Strahlungsdetektor (104) wenigstens ausgibt:

- ◦ die erste laterale Vorabansicht;
- ◦ die zweite laterale Vorabansicht;

- wobei sowohl die frontale Multienergie-Vorabansicht als auch die laterale Multienergie-Vorabansicht während derselben einzelnen vorläufigen vertikalen Abtastung erzeugt werden.

5. Radiologisches Bildgebungsverfahren nach einem der vorhergehenden Ansprüche, wobei:

- das frontale Bild (3) durch Durchführen einer einzelnen vertikalen Abtastung eines stehenden Patienten entlang der vertikalen Abtastrichtung durch die frontale Strahlungsquelle (101) und durch den frontalen Strahlungsdetektor (102) erzeugt wird;
- das laterale Bild (3) durch Durchführen einer einzelnen vertikalen Abtastung eines stehenden Patienten entlang der vertikalen Abtastrichtung durch die laterale Strahlungsquelle (103) und durch den lateralen Strahlungsdetektor (104) erzeugt wird;
- wobei sowohl das frontale Bild als auch das laterale Bild während derselben einzelnen vertikalen Abtastung erzeugt werden.

6. Radiologisches Bildgebungsverfahren nach einem der vorhergehenden Ansprüche, wobei ein erster vorgegebener Energieschwellenwert gleich wie oder kleiner als ein zweiter vorgegebener Energieschwellenwert ist, vorzugsweise gleich wie der zweite vorgegebene Energieschwellenwert, und vorzugsweise wobei:

- der erste vorgegebene Energieschwellenwert gleich wie der zweite vorgegebene Energieschwellenwert ist;
- die frontalen und/oder die lateralen Multienergie-Vorabansichten (1) erzeugt werden, sodass der frontale und/oder der laterale Strahlungsdetektor (102, 104) wenigstens ausgibt:

- ◦ die erste frontale Vorabansicht;
- ◦ eine dritte frontale Vorabansicht, welche der Gesamtenergie entspricht, welche von dem frontalen Strahlungsdetektor (102) empfangen wird, welche als Gesamtenergie-Frontal-Vorabansicht bezeichnet wird,

  - ▪ wobei die zweite frontale Vorabansicht durch ein Subtrahieren der ersten frontalen Vorabansicht von der dritten frontalen Vorabansicht erhalten wird;

◦ und/oder die erste laterale Vorabansicht;
◦ und/oder eine dritte laterale Vorabansicht, welche der Gesamtenergie entspricht, welche von dem lateralen Strahlungsdetektor (104) empfangen wird, welche als Gesamtenergie-Lateral-Vorabansicht bezeichnet wird,

▪ wobei die zweite laterale Vorabansicht durch ein Subtrahieren der ersten lateralen Vorabansicht von der dritten lateralen Vorabansicht erhalten wird.

7. Radiologisches Bildgebungsverfahren nach einem der vorhergehenden Ansprüche, wobei:

- die erste(n) frontale(n) und/oder laterale(n) Vorabansicht(en) und die zweite(n) frontale(n) und/oder laterale(n) Vorabansicht(en) kombiniert und verarbeitet werden, um ein Patientenknochen-Dickenprofil entlang der vertikalen Abtastrichtung abzuschätzen (22);
- und/oder die erste frontale und/oder laterale Vorabansicht(en) und die zweite frontale und/oder laterale Vorabansicht(en) kombiniert und verarbeitet werden, um ein Patient-Weichgewebe-Dickenprofil entlang der vertikalen Abtastrichtung abzuschätzen (22);
- das frontale Bild durch Durchführen einer vertikalen Abtastung eines stehenden Patienten entlang der vertikalen Abtastrichtung durch die frontale Strahlungsquelle (101) und durch den frontalen Strahlungsdetektor (102) erzeugt wird, mit:

◦ einer Modulation einer Ansteuerstromstärke von wenigstens der frontalen Strahlungsquelle (101) entlang der vertikalen Abtastrichtung, abhängig von dem Patientenknochen-Dickenprofil und/oder von dem Patient-Weichgewebe-Dickenprofil entlang der vertikalen Abtastrichtung;
◦ und vorzugsweise auch der Modulation der Ansteuerspannungsstärke der frontalen Strahlungsquelle (101) entlang der vertikalen Abtastrichtung, abhängig von dem Patientenknochen-Dickenprofil und von dem Patient-Weichgewebe-Dickenprofil entlang der vertikalen Abtastrichtung;

- das laterale Bild (3) durch Durchführen einer vertikalen Abtastung eines stehenden Patienten entlang der vertikalen Abtastrichtung durch die laterale Strahlungsquelle (103) und durch den lateralen Strahlungsdetektor (104) erzeugt wird, mit:

◦ einer Modulation einer Ansteuerstromstärke von wenigstens der lateralen Strahlungsquelle (103) entlang der vertikalen Abtastrichtung, abhängig von dem Patientenknochen-Dickenprofil und/oder von dem Patient-Weichgewebe-Dickenprofil entlang der vertikalen Abtastrichtung;
◦ und vorzugsweise auch der Modulation einer Ansteuerspannungsstärke der lateralen Strahlungsquelle (103) entlang der vertikalen Abtastrichtung, abhängig von dem Patientenknochen-Dickenprofil und von dem Patient-Weichgewebe-Dickenprofil entlang der vertikalen Abtastrichtung;

- wobei sowohl das frontale Bild als auch das laterale Bild während derselben vertikalen Abtastung erzeugt werden.

8. Radiologisches Bildgebungsverfahren nach einem der vorhergehenden Ansprüche, wobei:

- entweder die Ansteuerstromstärkenmodulation(en) der frontalen und/oder der lateralen Strahlungsquelle (101, 103), ohne Spannungsstärkenmodulation der frontalen und/oder der lateralen Strahlungsquelle (101, 103), automatisch durchgeführt wird, um einen Kompromiss zu verbessern zwischen:

◦ Verringern der globalen Strahlendosis, welche ein Patient während der vertikalen Abtastung empfängt,
◦ und Nicht-Verschlechtern unterhalb eines gegebenen Kontrastschwellenwerts der lokalen Bildkontraste der identifizierten spezifischen Knochenlokalisation an verschiedenen Bildgebungspositionen entlang der vertikalen Abtastrichtung, für alle oder einen Teil von Patientendicken entlang der vertikalen Abtastrichtung, für das frontale und/oder das laterale Bild;

- oder sowohl die Ansteuerstromstärkenmodulation als auch die Ansteuerspannungsstärkenmodulation der frontalen und/oder der lateralen Strahlungsquelle (101, 103) simultan, vorzugsweise synchron, und automatisch, durchgeführt werden, um einen Kompromiss zu verbessern zwischen:

◦ Verringern der globalen Strahlendosis, welche ein Patient während der vertikalen Abtastung empfängt,

◦ und Erhöhen der lokalen Bildkontraste der identifizierten spezifischen Knochenlokalisation an verschiedenen Bildgebungspositionen entlang der vertikalen Abtastrichtung, bezogen auf lokale Bildkontraste der identifizierten spezifischen Knochenlokalisation an verschiedenen Bildgebungspositionen entlang der vertikalen Abtastrichtung bei gleicher globaler Strahlendosis, aber ohne jegliche Ansteuerstromstärkenmodulation oder Ansteuerspannungsstärkenmodulation, für alle oder einen Teil von Patientendicken entlang der vertikalen Abtastrichtung, für das frontale und/oder das laterale Bild;

und/oder wobei:

- entweder die Ansteuerstromstärkenmodulation(en) der frontalen und/oder der lateralen Strahlungsquelle (101, 103), ohne Spannungsstärkenmodulation der frontalen und/oder der lateralen Strahlungsquelle (101, 103), automatisch durchgeführt wird, um einen Kompromiss zu verbessern zwischen:

◦ Verringern der globalen Strahlendosis, welche ein Patient während der vertikalen Abtastung empfängt,
◦ und Verbessern des Kontrast-Rausch-Verhältnisses oder des Verhältnisses zwischen Kontrast-Rausch-Verhältnis und Quadratwurzel der globalen Strahlendosis der identifizierten spezifischen Knochenlokalisation an verschiedenen Bildgebungspositionen entlang der vertikalen Abtastrichtung, bezogen auf lokale Bildkontraste der identifizierten spezifischen Knochenlokalisation an verschiedenen Bildgebungspositionen entlang der vertikalen Abtastrichtung bei gleicher globaler Strahlendosis, aber ohne jegliche Ansteuerstromstärkenmodulation, für alle oder einen Teil von Patientendicken entlang der vertikalen Abtastrichtung, für das frontale und/oder das laterale Bild;

- oder sowohl die Ansteuerstromstärkenmodulation als auch die Ansteuerspannungsstärkenmodulation der frontalen und/oder der lateralen Strahlungsquelle (101, 103) simultan, vorzugsweise synchron, und automatisch, durchgeführt werden, um einen Kompromiss zu verbessern zwischen:

◦ Verringern der globalen Strahlendosis, welche ein Patient während der vertikalen Abtastung empfängt,
◦ und Verbessern des Kontrast-Rausch-Verhältnisses oder des Verhältnisses zwischen Kontrast-Rausch-Verhältnis und Quadratwurzel der globalen Strahlendosis der identifizierten spezifischen Knochenlokalisation an verschiedenen Bildgebungspositionen entlang der vertikalen Abtastrichtung, bezogen auf lokale Bildkontraste der identifizierten spezifischen Knochenlokalisation an verschiedenen Bildgebungspositionen entlang der vertikalen Abtastrichtung bei gleicher globaler Strahlendosis, aber ohne jegliche Ansteuerstromstärkenmodulation oder Ansteuerspannungsstärkenmodulation, für alle oder einen Teil von Patientendicken entlang der vertikalen Abtastrichtung, für das frontale und/oder das laterale Bild.

9. Radiologisches Bildgebungsverfahren nach einem der vorhergehenden Ansprüche, wobei:

- die Aufnahme der frontalen Multienergie-Vorabansicht mit wenigstens 2 Energie-Bins durchgeführt wird (1), oder mit wenigstens 3 Energie-Bins, oder mit wenigstens 6 Energie-Bins,

◦ und/oder mit höchstens 20 Energie-Bins, oder höchstens 15 Energie-Bins, oder höchstens 10 Energie-Bins;

- und/oder die Aufnahme der lateralen Multienergie-Vorabansicht mit wenigstens 2 Energie-Bins durchgeführt wird (1), oder mit wenigstens 3 Energie-Bins, oder mit wenigstens 6 Energie-Bins,

◦und/oder mit höchstens 20 Energie-Bins, oder höchstens 15 Energie-Bins, oder höchstens 10 Energie-Bins.

10. Radiologisches Bildgebungsverfahren nach einem der vorhergehenden Ansprüche, wobei:

- die erste und die zweite frontale Vorabansicht zu einer Multimaterialzerlegung mit wenigstens zwei Materialdicken-Vertikalprofilen verarbeitet werden,

◦ vorzugsweise, entweder eine Bimaterialzerlegung zwischen Al und PMMA oder eine Bimaterialzerlegung zwischen HA und $H_2O$;

- und/oder die erste und die zweite laterale Vorabansicht zu einer Multimaterialzerlegung mit wenigstens zwei Materialdicken-Vertikalvektoren verarbeitet werden,

    ◦ vorzugsweise, entweder eine Bimaterialzerlegung zwischen Al und PMMA oder eine Bimaterialzerlegung zwischen HA und $H_2O$.

11. Radiologisches Bildgebungsverfahren nach einem der vorhergehenden Ansprüche, wobei sowohl die Ansteuerstromstärkenmodulationen als auch die Ansteuerspannungsstärkemodulation der frontalen und/oder der lateralen Strahlungsquelle (101, 103) derart durchgeführt werden, dass ein Wert eines Signal-Rausch-Verhältnisses erreicht wird, welcher konstant ist und für die meisten der Bildgebungspositionen entlang der vertikalen Abtastrichtung gleich ist, vorzugsweise für alle Bildgebungspositionen entlang der vertikalen Abtastrichtung, für das frontale Bild und/oder für das laterale Bild, aber welcher zwei unterschiedliche Werte für jeweils das frontale Bild und für das laterale Bild annehmen kann.

12. Radiologisches Bildgebungsverfahren nach Anspruch 11, wobei, für jedes der frontalen und/oder der lateralen Bilder, der Signal-Rausch-Verhältnis-Wert konstant ist und für jedes verschiedene Patientenorgan, welches abgebildet werden soll, vorbestimmt ist,
und/oder wobei:

    - für ein frontales Bild einer Patienten-Wirbelsäule der Standard-Signal-Rausch-Verhältnis-Wert einer Anzahl von Röntgenphotonen entspricht, welche pro Detektorpixel empfangen werden, welche zwischen 50 und 70 umfasst ist, wobei der Bediener des radiologischen Bildgebungsverfahrens vorzugsweise die Möglichkeit hat, mittels eines manuellen Befehls, von diesem Standardwert um wenigstens -25 % oder +100 %, weiter vorzugsweise um wenigstens -50 % oder +200 %, abzuweichen;
    - und/oder für ein laterales Bild einer Patienten-Wirbelsäule der Standard-Signal-Rausch-Verhältnis-Wert einer Anzahl von Röntgenphotonen entspricht, welche pro Detektorpixel empfangen werden, welche zwischen 20 und 40 umfasst ist, wobei der Bediener des radiologischen Bildgebungsverfahrens vorzugsweise die Möglichkeit hat, mittels eines manuellen Befehls, von diesem Standardwert um wenigstens -25 % oder +100 %, weiter vorzugsweise um wenigstens -50 % oder +200 %, abzuweichen.

13. Radiologisches Bildgebungsverfahren nach einem der vorhergehenden Ansprüche, wobei:

    - Modulationen von sowohl Stromstärke als auch Spannungsstärke:

        ◦ sowohl Stromstärke als auch Spannungsstärke für größere Patientendicken simultanes erhöhen;
        ◦ sowohl Stromstärke und Spannungsstärke für kleinere Patientendicken simultanes verringern;
        ◦ wobei eine Stromstärkenänderungsrate langsamer ist als eine Spannungsstärkenänderungsrate.

14. Radiologisches Bildgebungsverfahren nach einem der vorhergehenden Ansprüche, wobei die Stromstärkenmodulation derart maximiert wird, dass auch die vertikale Abtastgeschwindigkeit an einem konstanten Wert maximiert wird.

15. Radiologisches Bildgebungsverfahren nach einem der vorhergehenden Ansprüche, wobei die Stromstärkenmodulation(en)-Rate einen vorbestimmten Schwellenwert von 5 mA pro Millisekunde nicht überschreitet, oder einen vorbestimmten Schwellenwert von 2 mA pro Millisekunde nicht überschreitet, oder einen vorbestimmten Schwellenwert von 1 mA pro Millisekunde nicht überschreitet,

    und/oder wobei die Stromstärkenmodulation(en) wenigstens von 20 mA bis 300 mA reicht/reichen, und vorzugsweise von 10 mA bis 400 mA,
    und/oder wobei die Spannungsstärkenmodulation(en) wenigstens von 60 kV bis 100 kV reicht/reichen, und vorzugsweise von 50 kV bis 130 kV,
    und/oder wobei der Wert der vertikalen Abtastgeschwindigkeit wenigstens von 8 cm/s bis 20 cm/s reicht, und vorzugsweise von 0,4 cm/s bis 35 cm/s.

16. Radiologisches Bildgebungsverfahren nach einem der vorhergehenden Ansprüche, wobei jede der frontalen und/oder der lateralen Vorabansicht(en) erzeugt wird (1), durch Durchführen einer vorläufigen vertikalen Abtastung eines stehenden Patienten entlang einer vertikalen Abtastrichtung mit einer reduzierten globalen Strahlendosis im Vergleich zu jedem der frontalen und lateralen Bilder, vor Erzeugen jedes der frontalen und lateralen Bilder, und vorzugsweise wobei die reduzierte globale Strahlendosis weniger als 10 % der globalen Strahlendosis beträgt, weiter vorzugsweise weniger als 5 % der globalen Strahlendosis.

**17.** Radiologisches Bildgebungsverfahren nach einem der vorhergehenden Ansprüche, wobei Pixel in der Vorabansicht zusammengefasst werden, vorzugsweise zu Zonen von N×N Pixeln, weiter vorzugsweise zu Zonen von 2×2 Pixeln bis 10×10 Pixeln, um abgebildete Zonen herzustellen.

**18.** Radiologisches Bildgebungsverfahren nach einem der vorhergehenden Ansprüche, wobei die Bilder oder die abgebildeten Zonen verarbeitet werden, um markante Punkte (60) zu identifizieren, welche wiederum verwendet werden, um das Dickenprofil/die Dickenprofile zu berechnen (22) und die spezifische Knochenlokalisierung eines stehenden Patienten entlang der vertikalen Abtastrichtung zu identifizieren (21).

**19.** Radiologisches Bildgebungsverfahren nach einem der vorhergehenden Ansprüche, wobei die zwei Strahlungsdetektoren (102, 104) jeweils mit den zwei Strahlungsquellen (101, 103) verbunden sind, wobei die zwei Strahlungsdetektoren (102, 104) zwei Photonenzähldetektoren (PCD) sind, wobei jeder mit einer automatischen Bildverarbeitungsfunktion verbunden ist, welche automatisch Bildgraustufen ausgleicht, unabhängig davon, welche Strahlendosis an der sensiblen Fläche des Strahlungsdetektors (102, 104) empfangen wird, um Antworten der Detektoren (102, 104) zu homogenisieren,
und/oder wobei die zwei Strahlungsdetektoren (102, 104) jeweils mit den zwei Strahlungsquellen (101, 103) verbunden sind, wobei die zwei Strahlungsdetektoren (102, 104) zwei Multienergie-Zähldetektoren sind, vorzugsweise zwei energieaufgelöste Photonenzähldetektoren (ERPCD - Energy Resolved Photon Counting Detectors).

**20.** Radiologisches Bildgebungsverfahren nach einem der vorhergehenden Ansprüche, wobei der zweite Energieschwellenwert derart gewählt ist, dass ein Bildkontrast für Bereiche mit geringerer Patientendicke entlang einer vertikalen Richtung mehr verbessert wird als für Bereiche mit größerer Patientendicke entlang der vertikalen Richtung, wobei der zweite Energieschwellenwert vorzugsweise zwischen 50 keV und 90 keV gewählt wird, vorzugsweise zwischen 60 keV und 80 keV, weiter vorzugsweise der zweite Energieschwellenwert bei 70 keV gewählt wird.

**21.** Radiologisches Bildgebungsverfahren nach einem der vorhergehenden Ansprüche, wobei:

- das frontale Bild und/oder das laterale Bild beide Monoenergie-Bilder sind, welche mit Spannungsstärkenmodulation(en) der frontalen Strahlungsquelle (101) und/oder der lateralen Strahlungsquelle (103) durchgeführt werden (3),
- oder das frontale Bild und/oder das laterale Bild beide Multienergie-Bilder sind, welche ohne Spannungsstärkenmodulation der frontalen Strahlungsquelle (101) und/oder der lateralen Strahlungsquelle (103) durchgeführt werden.

**Revendications**

**1.** Procédé d'imagerie radiologique comprenant :

> 2 sources de rayonnement (101, 103) avec des directions d'imagerie orthogonales l'une à l'autre, une source de rayonnement frontale (101) et une source de rayonnement latérale (103), coulissant verticalement de manière à effectuer un balayage vertical d'un patient debout le long d'une direction de balayage vertical,
> 2 détecteurs de rayonnement (102, 104) qui sont respectivement associés auxdites 2 sources de rayonnement (101, 103), un détecteur de rayonnement frontal (102) et un détecteur de rayonnement latéral (104), coulissant verticalement de manière à effectuer un balayage vertical d'un patient debout le long de ladite direction de balayage vertical, au moins ledit détecteur de rayonnement frontal (102, 104) étant un détecteur à comptage multi-énergie,

dans lequel ledit procédé radiologique comprend au moins un mode de fonctionnement dans lequel :

≻ une vue de reconnaissance frontale multi-énergie est réalisé (1) en effectuant un balayage vertical préliminaire d'un patient debout le long de ladite direction de balayage vertical par ladite source de rayonnement frontale (101) et par ledit détecteur de rayonnement frontal (102), de sorte que ledit détecteur de rayonnement frontal (102) produise au moins :

∘ une première vue de reconnaissance frontale correspondant à une première portion d'énergie qui est reçue par ledit détecteur de rayonnement frontal (102) et qui est au-dessous d'un premier seuil d'énergie donné, appelée vue de reconnaissance frontale à faible énergie,

◦ une deuxième vue de reconnaissance frontale correspondant à une seconde portion d'énergie qui est reçue par ledit détecteur de rayonnement frontal (102) et qui est au-dessus d'un second seuil d'énergie donné, appelée vue de reconnaissance frontale à haute énergie,

➢ ladite première vue de reconnaissance frontale et ladite deuxième vue de reconnaissance frontale sont combinées et traitées (20) de manière à évaluer :

◦ au moins une épaisseur osseuse (22) de patient,
◦ au moins une épaisseur de tissu mou (22) de patient,
◦ une localisation osseuse spécifique au patient à différentes positions d'imagerie le long de ladite direction de balayage vertical (21),

➢ une image frontale est réalisée (3) en effectuant un balayage vertical d'un patient debout le long de ladite direction de balayage vertical par ladite source de rayonnement frontale (101) et par ledit détecteur de rayonnement frontal (102), avec :

◦ une modulation d'une intensité de courant d'excitation d'au moins ladite source de rayonnement frontale (101) le long de ladite direction de balayage vertical, en fonction de ladite épaisseur osseuse de patient, de ladite épaisseur de tissu mou de patient, et de ladite localisation osseuse spécifique au patient à différentes positions d'imagerie le long de ladite direction de balayage vertical,
◦ et de préférence également une modulation d'une intensité de tension d'excitation de ladite source de rayonnement frontale (101) le long de ladite direction de balayage vertical, en fonction de ladite épaisseur osseuse de patient, de ladite épaisseur de tissu mou de patient, et de ladite localisation osseuse spécifique au patient à différentes positions d'imagerie le long de ladite direction de balayage vertical,
◦ soit une modulation d'intensité de courant d'excitation de ladite source de rayonnement frontale (101), sans aucune modulation d'intensité de tension de ladite source de rayonnement frontale (101), est effectuée automatiquement, de manière à améliorer un compromis entre :

▪ la dose de rayonnement globale reçue par un patient lors dudit balayage vertical,
▪ et les contrastes d'image locaux de ladite localisation osseuse spécifique identifiée à différentes positions d'imagerie le long de ladite direction de balayage vertical, pour l'image frontale,

◦ soit les deux modulations d'intensité de courant et d'intensité de tension d'excitation de ladite source de rayonnement frontale (101) sont effectuées simultanément, de préférence de manière synchrone, et automatiquement, de manière à améliorer un compromis entre :

▪ la dose de rayonnement globale reçue par un patient lors dudit balayage vertical,
▪ et les contrastes d'image locaux de ladite localisation osseuse spécifique identifiée à différentes positions d'imagerie le long de ladite direction de balayage vertical, pour l'image frontale.

2. Procédé d'imagerie radiologique comprenant :

> 2 sources de rayonnement (101, 103) avec des directions d'imagerie orthogonales l'une à l'autre, une source de rayonnement frontale (101) et une source de rayonnement latérale (103), coulissant verticalement de manière à effectuer un balayage vertical d'un patient debout le long d'une direction de balayage vertical,
> 2 détecteurs de rayonnement (102, 104) qui sont respectivement associés auxdites 2 sources de rayonnement (101, 103), un détecteur de rayonnement frontal (102) et un détecteur de rayonnement latéral (104), coulissant verticalement de manière à effectuer un balayage vertical d'un patient debout le long de ladite direction de balayage vertical, au moins ledit détecteur de rayonnement latéral (104) étant un détecteur à comptage multi-énergie,

dans lequel ledit procédé radiologique comprend au moins un mode de fonctionnement dans lequel :

➢ une vue de reconnaissance latérale multi-énergie est réalisée (1) en effectuant un balayage vertical préliminaire d'un patient debout le long de ladite direction de balayage vertical par ladite source de rayonnement latérale (103) et par ledit détecteur de rayonnement latéral (104), de sorte que ledit détecteur de rayonnement latéral (104) produise au moins :

◦ une première vue de reconnaissance latérale correspondant à une première portion d'énergie qui est reçue par ledit détecteur de rayonnement latéral (104) et qui est au-dessous d'un premier seuil d'énergie donné, appelée vue de reconnaissance latérale à faible énergie,

◦ une deuxième vue de reconnaissance latérale correspondant à une seconde portion d'énergie qui est reçue par ledit détecteur de rayonnement latéral (104) et qui est au-dessus d'un second seuil d'énergie donné, appelée vue de reconnaissance latérale à haute énergie,

➢ ladite première vue de reconnaissance latérale et ladite deuxième vue de reconnaissance latérale sont combinées et traitées (20) de manière à évaluer :

◦ au moins une épaisseur osseuse (22) de patient,
◦ au moins une épaisseur de tissu mou (22) de patient,
◦ une localisation osseuse spécifique au patient à différentes positions d'imagerie le long de ladite direction de balayage vertical (21),

➢ une image latérale est réalisée (3) en effectuant un balayage vertical d'un patient debout le long de ladite direction de balayage vertical par ladite source de rayonnement latérale (103) et par ledit détecteur de rayonnement latéral (104), avec :

◦ une modulation d'une intensité de courant d'excitation d'au moins ladite source de rayonnement latérale (103) le long de ladite direction de balayage vertical, en fonction de ladite épaisseur osseuse de patient, de ladite épaisseur de tissu mou de patient, et de ladite localisation osseuse spécifique au patient à différentes positions d'imagerie le long de ladite direction de balayage vertical,

◦ et de préférence également une modulation d'une intensité de tension d'excitation de ladite source de rayonnement latérale (103) le long de ladite direction de balayage vertical, en fonction de ladite épaisseur osseuse de patient, de ladite épaisseur de tissu mou de patient, et de ladite localisation osseuse spécifique au patient à différentes positions d'imagerie le long de ladite direction de balayage vertical,

◦ soit une modulation d'intensité de courant d'excitation de ladite source de rayonnement latérale (103), sans aucune modulation d'intensité de tension de ladite source de rayonnement latérale (103), est effectuée automatiquement, de manière à améliorer un compromis entre :

■ la dose de rayonnement globale reçue par un patient lors dudit balayage vertical,
■ et les contrastes d'image locaux de ladite localisation osseuse spécifique identifiée à différentes positions d'imagerie le long de ladite direction de balayage vertical, pour l'image latérale,

◦ soit les deux modulations d'intensité de courant et d'intensité de tension d'excitation de ladite source de rayonnement latérale (103) sont effectuées simultanément, de préférence de manière synchrone, et automatiquement, de manière à améliorer un compromis entre :

■ la dose de rayonnement globale reçue par un patient lors dudit balayage vertical,
■ et les contrastes d'image locaux de ladite localisation osseuse spécifique identifiée à différentes positions d'imagerie le long de ladite direction de balayage vertical, pour l'image latérale.

3. Procédé d'imagerie radiologique comprenant :

> 2 sources de rayonnement (101, 103) avec des directions d'imagerie orthogonales l'une à l'autre, une source de rayonnement frontale (101) et une source de rayonnement latérale (103), coulissant verticalement de manière à effectuer un balayage vertical d'un patient debout le long d'une direction de balayage vertical,

> 2 détecteurs de rayonnement (102, 104) qui sont respectivement associés auxdites 2 sources de rayonnement (101, 103), un détecteur de rayonnement frontal (102) et un détecteur de rayonnement latéral (104), coulissant verticalement de manière à effectuer un balayage vertical d'un patient debout le long de ladite direction de balayage vertical, lesdits 2 détecteurs de rayonnement (102, 104) étant respectivement 2 détecteurs à comptage multi-énergie,

dans lequel ledit procédé radiologique comprend au moins un mode de fonctionnement dans lequel :

➢ des vues de reconnaissance frontale et latérale multi-énergie sont réalisées (1) en effectuant un balayage vertical préliminaire d'un patient debout le long de ladite direction de balayage vertical par lesdites sources de rayonnement frontale et latérale (101, 103) et par lesdits détecteurs de rayonnement frontal et latéral (102, 104), de sorte que lesdits détecteurs de rayonnement frontal et latéral (102, 104) produisent au moins :

◦ une première vue de reconnaissance frontale correspondant à une première portion d'énergie qui est reçue par ledit détecteur de rayonnement frontal (102) et qui est au-dessous d'un premier seuil d'énergie donné, appelée vue de reconnaissance frontale à faible énergie,
◦ une deuxième vue de reconnaissance frontale correspondant à une seconde portion d'énergie qui est reçue par ledit détecteur de rayonnement frontal (102) et qui est au-dessus d'un second seuil d'énergie donné, appelée vue de reconnaissance frontale à haute énergie,
◦ une première vue de reconnaissance latérale correspondant à une première portion d'énergie qui est reçue par ledit détecteur de rayonnement latéral (104) et qui est au-dessous d'un premier seuil d'énergie donné, appelée vue de reconnaissance latérale à faible énergie,
◦ une deuxième vue de reconnaissance latérale correspondant à une seconde portion d'énergie qui est reçue par ledit détecteur de rayonnement latéral (104) et qui est au-dessus d'un second seuil d'énergie donné, appelée vue de reconnaissance latérale à haute énergie,

➢ lesdites premières vues de reconnaissance frontale et latérale et lesdites deuxièmes vues de reconnaissance frontale et latérale sont combinées et traitées (20) de manière à évaluer :

◦ au moins une épaisseur osseuse (22) de patient,
◦ au moins une épaisseur de tissu mou (22) de patient,
◦ une localisation osseuse spécifique au patient à différentes positions d'imagerie le long de ladite direction de balayage vertical (21),

➢ une image frontale est réalisée (3) en effectuant un balayage vertical d'un patient debout le long de ladite direction de balayage vertical par ladite source de rayonnement frontale (101) et par ledit détecteur de rayonnement frontal (102), et une image latérale est réalisée en effectuant un balayage vertical d'un patient debout le long de ladite direction de balayage vertical par ladite source de rayonnement latérale (103) et par ledit détecteur de rayonnement latéral (104), les images frontale et latérale étant toutes deux réalisées lors d'un même balayage vertical, avec :

◦ une modulation d'intensités de courant d'excitation desdites deux sources de rayonnement frontale et latérale (101, 103) le long de ladite direction de balayage vertical, en fonction de ladite épaisseur osseuse de patient, de ladite épaisseur de tissu mou de patient, et de ladite localisation osseuse spécifique au patient à différentes positions d'imagerie le long de ladite direction de balayage vertical,
◦ et de préférence également une modulation d'intensités de tension d'excitation desdites deux sources de rayonnement frontale et latérale (101, 103) le long de ladite direction de balayage vertical, en fonction de ladite épaisseur osseuse de patient, de ladite épaisseur de tissu mou de patient, et de ladite localisation osseuse spécifique au patient à différentes positions d'imagerie le long de ladite direction de balayage vertical,
◦ soit une modulation d'intensité de courant d'excitation de ladite source de rayonnement frontale (101), sans aucune modulation d'intensité de tension de ladite source de rayonnement frontale (101), ainsi qu'une modulation d'intensité de courant d'excitation de ladite source de rayonnement latérale (103), sans aucune modulation d'intensité de tension de ladite source de rayonnement latérale (103), sont effectuées simultanément, de préférence de manière synchrone, et automatiquement, de manière à améliorer un compromis entre :

▪ la dose de rayonnement globale reçue par un patient lors dudit balayage vertical,
▪ et les contrastes d'image locaux de ladite localisation osseuse spécifique identifiée à différentes positions d'imagerie le long de ladite direction de balayage vertical, pour l'image frontale et pour l'image latérale,

◦ soit les deux modulations d'intensité de courant et d'intensité de tension d'excitation de ladite source de rayonnement frontale (101), sont effectuées simultanément, de préférence de manière synchrone, et automatiquement, ainsi que les deux modulations d'intensité de courant et d'intensité de tension d'excitation de ladite source de rayonnement latérale (103), sont effectuées simultanément, de préférence de manière synchrone, et automatiquement, de manière à améliorer un compromis entre :

- la dose de rayonnement globale reçue par un patient lors dudit balayage vertical,
- et les contrastes d'image locaux de ladite localisation osseuse spécifique identifiée à différentes positions d'imagerie le long de ladite direction de balayage vertical, pour l'image frontale et pour l'image latérale.

4. Procédé d'imagerie radiologique selon l'une quelconque des revendications précédentes, dans lequel :

➢ ladite vue de reconnaissance frontale multi-énergie est réalisée (1) en effectuant un balayage vertical préliminaire unique d'un patient debout le long de ladite direction de balayage vertical par ladite source de rayonnement frontale (101) et par ledit détecteur de rayonnement frontal (102), de sorte que ledit détecteur de rayonnement frontal (102) produise au moins :

  ◦ ladite première vue de reconnaissance frontale,
  ◦ ladite deuxième vue de reconnaissance frontale,

➢ ladite vue de reconnaissance latérale multi-énergie est réalisée (1) en effectuant un balayage vertical préliminaire unique d'un patient debout le long de ladite direction de balayage vertical par ladite source de rayonnement latérale (103) et par ledit détecteur de rayonnement latéral (104), de sorte que ledit détecteur de rayonnement latéral (104) produise au moins :

  ◦ ladite première vue de reconnaissance latérale,
  ◦ ladite deuxième vue de reconnaissance latérale,

➢ ladite vue de reconnaissance frontale multi-énergie et ladite vue de reconnaissance latérale multi-énergie étant tous deux réalisées lors d'un même balayage vertical préliminaire unique.

5. Procédé d'imagerie radiologique selon l'une quelconque des revendications précédentes, dans lequel :

➢ ladite image frontale est réalisée (3) en effectuant un balayage vertical unique d'un patient debout le long de ladite direction de balayage vertical par ladite source de rayonnement frontale (101) et par ledit détecteur de rayonnement frontal (102),
➢ ladite image latérale est réalisée (3) en effectuant un balayage vertical unique d'un patient debout le long de ladite direction de balayage vertical par ladite source de rayonnement latérale (103) et par ledit détecteur de rayonnement latéral (104),
➢ ladite image frontale et ladite image latérale étant toutes deux réalisées lors d'un même balayage vertical unique.

6. Procédé d'imagerie radiologique selon l'une quelconque des revendications précédentes, dans lequel ledit premier seuil d'énergie donné est égal ou inférieur audit second seuil d'énergie donné, de préférence égal audit second seuil d'énergie donné, et de préférence dans lequel :

➢ ledit premier seuil d'énergie donné est égal audit second seuil d'énergie donné,
➢ lesdites vues de reconnaissance frontale et/ou latérale multi-énergie sont réalisées (1) de sorte que lesdits détecteurs de rayonnements frontal et/ou latéral (102, 104) produisent tout d'abord :

  ◦ ladite première vue de reconnaissance frontale,
  ◦ une troisième vue de reconnaissance frontale correspondant à l'énergie entière qui est reçue par ledit détecteur de rayonnement frontal (102), appelée vue de reconnaissance frontale d'énergie totale,

    - ladite deuxième vue de reconnaissance frontale étant obtenue en soustrayant ladite première vue de reconnaissance frontale de ladite troisième vue de reconnaissance frontale,

  ◦ et/ou ladite première vue de reconnaissance latérale,
  ◦ et/ou une troisième vue de reconnaissance latérale correspondant à l'énergie entière qui est reçue par ledit détecteur de rayonnement latéral (104), appelée vue de reconnaissance latérale d'énergie totale,

    - ladite deuxième vue de reconnaissance latérale étant obtenue en soustrayant ladite première vue de reconnaissance latérale de ladite troisième vue de reconnaissance latérale.

7. Procédé d'imagerie radiologique selon l'une quelconque des revendications précédentes, dans lequel :

➢ ladite ou lesdites premières vues de reconnaissance frontale et/ou latérale et ladite ou lesdites deuxièmes vues de reconnaissance frontale et/ou latérale sont combinées et traitées de manière à évaluer (22) un profil d'épaisseur osseuse de patient le long de ladite direction de balayage vertical,

➢ et/ou ladite ou lesdites premières vues de reconnaissance frontale et/ou latérale et ladite ou lesdites deuxièmes vues de reconnaissance frontale et/ou latérale sont combinées et traitées de manière à évaluer (22) un profil d'épaisseur de tissu mou de patient le long de ladite direction de balayage vertical,

➢ ladite image frontale est réalisée en effectuant un balayage vertical d'un patient debout le long de ladite direction de balayage vertical par ladite source de rayonnement frontale (101) et par ledit détecteur de rayonnement frontal (102), avec :

○ une modulation d'une intensité de courant d'excitation d'au moins ladite source de rayonnement frontale (101) le long de ladite direction de balayage vertical, en fonction dudit profil d'épaisseur osseuse de patient et/ou dudit profil d'épaisseur de tissu mou de patient le long de ladite direction de balayage vertical,

○ et de préférence également ladite modulation d'une intensité de tension d'excitation de ladite source de rayonnement frontale (101) le long de ladite direction de balayage vertical, en fonction dudit profil d'épaisseur osseuse de patient et dudit profil d'épaisseur de tissu mou de patient le long de ladite direction de balayage vertical,

➢ ladite image latérale est réalisée en effectuant un balayage vertical d'un patient debout le long de ladite direction de balayage vertical par ladite source de rayonnement latérale (103) et par ledit détecteur de rayonnement latéral (104), avec :

○ une modulation d'une intensité de courant d'excitation d'au moins ladite source de rayonnement latérale (103) le long de ladite direction de balayage vertical, en fonction dudit profil d'épaisseur osseuse de patient et/ou dudit profil d'épaisseur de tissu mou de patient le long de ladite direction de balayage vertical,

○ et de préférence également ladite modulation d'une intensité de tension d'excitation de ladite source de rayonnement latérale (103) le long de ladite direction de balayage vertical, en fonction dudit profil d'épaisseur osseuse de patient et dudit profil d'épaisseur de tissu mou de patient le long de ladite direction de balayage vertical,

➢ ladite image frontale et ladite image latérale étant toutes deux réalisées lors d'un même balayage vertical.

8. Procédé d'imagerie radiologique selon l'une quelconque des revendications précédentes, dans lequel :

○ soit ladite ou lesdites modulations d'intensité de courant d'excitation de ladite ou desdites sources de rayonnement frontale et/ou latérale (101, 103), sans aucune modulation d'intensité de tension de ladite ou desdites sources de rayonnement frontale et/ou latérale (101, 103), sont effectuées automatiquement, de manière à améliorer un compromis entre :

▪ la diminution de la dose de rayonnement globale reçue par un patient lors dudit balayage vertical,

▪ et la non-dégradation, sous un seuil de contraste donné, des contrastes d'image locaux de ladite localisation osseuse spécifique identifiée à différentes positions d'imagerie le long de ladite direction de balayage vertical, pour tout ou partie d'épaisseurs de patient le long de ladite direction de balayage vertical, pour la ou les images frontale et/ou latérale,

○ ou lesdites deux modulations d'intensité de courant et d'intensité de tension d'excitation de ladite ou desdites sources de rayonnement frontale et/ou latérale (101, 103) sont effectuées simultanément, de préférence de manière synchrone, et automatiquement, de manière à améliorer un compromis entre :

▪ la diminution de la dose de rayonnement globale reçue par un patient lors dudit balayage vertical,

▪ et l'augmentation des contrastes d'image locaux de la localisation osseuse spécifique identifiée à différentes positions d'imagerie le long de ladite direction de balayage vertical, par rapport à des contrastes d'image locaux de ladite localisation osseuse spécifique identifiée à différentes positions d'imagerie le long de ladite direction de balayage vertical avec une même dose de rayonnement globale, mais sans aucune modulation d'intensité de courant d'excitation ni aucune modulation d'intensité de tension d'excitation, pour tout ou partie d'épaisseurs de patient le long de ladite direction de balayage vertical, pour la ou les images frontale et/ou latérale,

et/ou dans lequel :

◦ soit ladite ou lesdites modulations d'intensité de courant d'excitation de ladite ou desdites sources de rayonnement frontale et/ou latérale (101, 103), sans aucune modulation d'intensité de tension de ladite ou desdites sources de rayonnement frontale et/ou latérale (101, 103), sont effectuées automatiquement, de manière à améliorer un compromis entre :

- la diminution de la dose de rayonnement globale reçue par un patient lors dudit balayage vertical,
- et l'amélioration du rapport contraste/bruit ou du rapport entre le rapport contraste/bruit et la racine carrée de ladite dose de rayonnement globale de ladite localisation osseuse spécifique identifiée à différentes positions d'imagerie le long de ladite direction de balayage vertical, par rapport à des contrastes d'image locaux de ladite localisation osseuse spécifique identifiée à différentes positions d'imagerie le long de ladite direction de balayage vertical avec une même dose de rayonnement globale, mais sans aucune modulation d'intensité de courant d'excitation, pour tout ou partie d'épaisseurs de patient le long de ladite direction de balayage vertical, pour la ou les images frontale et/ou latérale,

◦ soit lesdites deux modulations d'intensité de courant et d'intensité de tension d'excitation de ladite ou desdites sources de rayonnement frontale et/ou latérale (101, 103) sont effectuées simultanément, de préférence de manière synchrone, et automatiquement, de manière à améliorer un compromis entre :

- la diminution de la dose de rayonnement globale reçue par un patient lors dudit balayage vertical,
- et l'amélioration du rapport contraste/bruit ou du rapport entre le rapport contraste/bruit et la racine carrée de ladite dose de rayonnement globale de ladite localisation osseuse spécifique identifiée à différentes positions d'imagerie le long de ladite direction de balayage vertical, par rapport à des contrastes d'image locaux de ladite localisation osseuse spécifique identifiée à différentes positions d'imagerie le long de ladite direction de balayage vertical avec une même dose de rayonnement globale, mais sans aucune modulation d'intensité de courant d'excitation ni aucune modulation d'intensité de tension d'excitation, pour tout ou partie d'épaisseurs de patient le long de ladite direction de balayage vertical, pour la ou les images frontale et/ou latérale.

9. Procédé d'imagerie radiologique selon l'une quelconque des revendications précédentes, dans lequel :

➢ ladite acquisition d'une vue de reconnaissance frontale multi-énergie est effectuée (1) avec au moins 2 bandes d'énergie, ou avec au moins 3 bandes d'énergie, ou avec au moins 6 bandes d'énergie,

◦ et/ou au plus 20 bandes d'énergie, ou au plus 15 bandes d'énergie, ou au plus 10 bandes d'énergie,

> et/ou ladite acquisition d'une vue de reconnaissance latérale multi-énergie est effectuée (1) avec au moins 2 bandes d'énergie, ou avec au moins 3 bandes d'énergie, ou avec au moins 6 bandes d'énergie,

◦ et/ou au plus 20 bandes d'énergie, ou au plus 15 bandes d'énergie, ou au plus 10 bandes d'énergie.

10. Procédé d'imagerie radiologique selon l'une quelconque des revendications précédentes, dans lequel :

➢ lesdites première et deuxième vues de reconnaissance frontales sont traitées en une décomposition multi-matériau avec au moins deux profils verticaux d'épaisseur de matériau,

◦ de préférence, soit une décomposition bi-matériau entre Al et PMMA, soit une décomposition bi-matériau entre HA et $H_2O$,

> et/ou lesdites première et deuxième vues de reconnaissance latérales sont traitées en une décomposition multi-matériau avec au moins deux vecteurs verticaux d'épaisseur de matériau,

◦ de préférence. soit une décomposition bi-matériau entre Al et PMMA, soit une décomposition bi-matériau entre HA et $H_2O$.

11. Procédé d'imagerie radiologique selon l'une quelconque des revendications précédentes, dans lequel lesdites deux modulations d'intensité de courant et d'intensité de tension d'excitation de ladite ou desdites sources de rayonnement frontale et/ou latérale (101, 103) sont également effectuées de manière à atteindre une valeur de rapport signal/bruit qui est constante et commune à la plupart desdites positions d'imagerie le long de ladite direction de balayage vertical, de préférence à toutes lesdites positions d'imagerie le long de ladite direction de balayage vertical, pour ladite image frontale et/ou pour ladite image latérale, mais qui peut prendre deux valeurs différentes pour l'image frontale et pour l'image latérale respectivement.

12. Procédé d'imagerie radiologique selon la revendication 11, dans lequel, pour chacune desdites images frontale et/ou latérale, ladite valeur de rapport signal/bruit est constante et prédéterminée pour chaque organe de patient différent à imager,
et/ou dans lequel :

➢ pour une image frontale d'une colonne vertébrale de patient, ladite valeur de rapport signal/bruit standard correspond à un nombre de photons de rayons X reçus par pixel détecteur compris entre 50 et 70, l'opérateur de procédé d'imagerie radiologique ayant de préférence la possibilité de s'écarter, via une commande manuelle, de cette valeur standard d'au moins - 25 % ou + 100 %, de manière davantage préférée d'au moins - 50 % ou + 200 %,
➢ et/ou pour une image frontale d'une colonne vertébrale de patient, ladite valeur de rapport signal/bruit standard correspond à un nombre de photons de rayons X reçus par pixel détecteur compris entre 20 et 40, l'opérateur de procédé d'imagerie radiologique ayant de préférence la possibilité de s'écarter, via une commande manuelle, de cette valeur standard d'au moins - 25 % ou + 100 %, de manière davantage préférée d'au moins - 50 % ou + 200 %.

13. Procédé d'imagerie radiologique selon l'une quelconque des revendications précédentes, dans lequel :

➢ des modulations à la fois de l'intensité de courant et de l'intensité de tension :

◦ augmentent simultanément à la fois l'intensité de courant et l'intensité de tension pour de plus importantes épaisseurs de patient,
◦ diminuent simultanément à la fois l'intensité de courant et l'intensité de tension pour de plus petites épaisseurs de patient,
◦ un taux de variation d'intensité de courant étant plus faible qu'un taux de variation d'intensité de tension.

14. Procédé d'imagerie radiologique selon l'une quelconque des revendications précédentes, dans lequel ladite modulation d'intensité de courant est optimisée de manière à aussi optimiser ladite vitesse de balayage vertical à une valeur constante.

15. Procédé d'imagerie radiologique selon l'une quelconque des revendications précédentes, dans lequel ledit taux de modulation(s) d'intensité de courant ne dépasse pas un seuil prédéterminé de 5 mA par milliseconde, ou ne dépasse pas un seuil prédéterminé de 2 mA par milliseconde, ou ne dépasse pas un seuil prédéterminé de 1 mA par milliseconde,

et/ou dans lequel ladite ou lesdites modulations d'intensité de courant vont au moins de 20 mA à 300 mA, et de préférence de 10 mA à 400 mA,
et/ou dans lequel ladite ou lesdites modulations d'intensité de tension vont au moins de 60 kV à 100 kV, et de préférence de 50 kV à 130 kV,
et/ou dans lequel ladite valeur de vitesse de balayage vertical va au moins de 8 cm/seconde à 20 cm/seconde, et de préférence de 0,4 cm/seconde à 35 cm/seconde.

16. Procédé d'imagerie radiologique selon l'une quelconque des revendications précédentes, dans lequel chacune de ladite ou desdites vues de reconnaissance frontale et/ou latérale est réalisée (1) en effectuant un balayage vertical préliminaire d'un patient debout le long d'une direction de balayage vertical avec une dose de rayonnement globale réduite par rapport à chacune desdites images frontale et latérale, avant de réaliser chacune desdites images frontale et latérale, et de préférence dans lequel ledit rayonnement global réduit est inférieur à 10 % de ladite dose de rayonnement globale, de manière davantage préférée inférieure à 5 % de ladite dose de rayonnement globale.

17. Procédé d'imagerie radiologique selon l'une quelconque des revendications précédentes, dans lequel des pixels dans ladite vue de reconnaissance sont rassemblés, de préférence par zones de NxN pixels, de manière davantage préférée par zones allant de 2x2 pixels à 10x10 pixels, pour réaliser des zones imagées.

18. Procédé d'imagerie radiologique selon l'une quelconque des revendications précédentes, dans lequel lesdites images ou lesdites zones imagées sont traitées pour identifier des points saillants (60) qui sont à leur tour utilisés pour calculer (22) ledit ou lesdits profil d'épaisseur et pour identifier (21) ladite localisation osseuse spécifique d'un patient debout le long de ladite direction de balayage vertical.

19. Procédé d'imagerie radiologique selon l'une quelconque des revendications précédentes, dans lequel lesdits 2 détecteurs de rayonnement (102, 104) sont respectivement associés auxdites 2 sources de rayonnement (101, 103), lesdits 2 détecteurs de rayonnement (102, 104) étant 2 détecteurs à comptage de photons (PCD) associés chacun à une fonction de traitement d'image automatique équilibrant automatiquement des niveaux de gris d'image quelle que soit la dose de rayonnement reçue sur la surface sensible dudit détecteur de rayonnement (102, 104) pour homogénéiser des réponses desdits détecteurs (102, 104),
et/ou dans lequel lesdits 2 détecteurs de rayonnement (102, 104) sont respectivement associés auxdites 2 sources de rayonnement (101, 103), lesdits 2 détecteurs de rayonnement (102, 104) étant 2 détecteurs à comptage multi-énergie, de préférence étant 2 détecteurs à comptage de photons à résolution énergétique (ERPCD).

20. Procédé d'imagerie radiologique selon l'une quelconque des revendications précédentes, dans lequel ledit second seuil d'énergie est choisi de manière à améliorer un contraste d'image davantage pour des régions d'épaisseurs de patient inférieures le long de la direction verticale que pour des régions d'épaisseurs de patient supérieures le long de la direction verticale, de préférence ledit second seuil d'énergie étant choisi entre 50 keV et 90 keV, de préférence entre 60 keV et 80 keV, de manière davantage préférée ledit second seuil d'énergie étant choisi à 70 keV.

21. Procédé d'imagerie radiologique selon l'une quelconque des revendications précédentes, dans lequel :

> ladite image frontale et/ou ladite image latérale sont toutes deux des images monoénergie, effectuées (3) avec ladite ou lesdites modulations d'intensité de tension de ladite source de rayonnement frontale (101) et/ou de ladite source de rayonnement latérale (103),
> ou ladite image frontale et/ou ladite image latérale sont toutes deux des images multi-énergie, effectuées sans aucune modulation d'intensité de tension, de ladite source de rayonnement frontale (101) et/ou de ladite source de rayonnement latérale (103).

2

**Exposure parameter computation**

1 — Multi-energy SCOUT acquisition

20 — **Thickness profile extraction**

24

21 — Bone localization computation (profile-of-interest extraction)

22 — Patient bone and soft-tissue thickness computation

*Bone and soft-tissue thickness profiles*

Model / Reference table

23 — Fixed kV value and Spectral filter, and detector energy threshold selection from Model / Reference table

*Spectral filtration*
*Fixed kV value*
*Detector energy threshold(s)*

*Feedback loop*

25 — Exposure target

26 — Acquisition speed & modulated mA profile computation

*Spectral filtration*
*kV value*
*Detector energy threshold(s)*
*Acquisition speed (mm/s)*
*mA modulation profile*

3 — Single- or multi-energy IMAGE acquisition

4 — IMAGE normalization

5 — IMAGE processing & display

Fig 1

Fig 2

Fig 4

Attenuated spectrum

keV

Total-energy bin

High-energy bin

Fig 3

Attenuated spectrum

keV

Low-energy bin

High-energy bin

Fig. 9

EP 4 525 724 B1

Fig 6

**Offline signal-to-thickness mapping calibration**

SCOUT exposure parameters

Known material thicknesses

Measurements or simulations

Detector signals

Saved signal-to-thickness mappings

Fig 7

**Online thickness computation**

Detector signals

Saved signal-to-**bone** mapping

Bone thickness

Saved signal-to-**soft-tissues** mapping

Soft-tissue thickness

EP 4 525 724 B1

Fig. 9

Fig. 10

Fig. 8

52

Fig 16

Fig 15

Fig 14

Fig 13

Fig 12

Fig 11

Thickness profiles (2E)

Fig 1B

Thickness profiles (1E)

Fig 1A

Exposure Control output parameters @ 45.0 mm/s (1E)

**Tube power**

Power limit (42 kW)
FRT
LAT

**Tube current**

mA limits
FRT
LAT

**Tube peak kilovoltage**

KVp limits
FRT (fixed kV = 120 kV)
LAT (fixed kV = 120 kV)

FRT

LAT

Expected tube power profiles

Computed mA profiles

Computed fixed kV values

Fig 19

Fig 20

Fig 21

Fig 22

Fig 2.

Exposure Control output parameters @ 45.0 mm/s (1E)

Tube power

P

Power limit (42 kW)
FRT
LAT

370
372
374

mA

I

Tube current

mA limits
FRT
LAT

400
350
300
250
200
150
100
50

Distance from top (cm)

380
382
381

Tube peak kilovoltage

V

kVp limits
FRT
LAT

130
120
110
100
90
80
70
60
50

390
392
391

FRT

LAT

Distance from top (cm)

Distance from top (cm)

Expected tube power profiles

Computed mA profiles

Computed modulated kV profiles

EP 4 525 724 B1

Fig 24

Fig 25

Fig 26

Fig 27

Fig 28

Exposure Control output parameters @ 45.0 mm/s (2E)

Tube power

Tube current

Tube peak kilovoltage

Expected tube power profiles

Computed mA profiles

Computed modulated kV profiles

Fig 29

Fig 30

Fig 31

Fig 32

Fig 33

Fig 34

Fig 35

Fig 36

Fig 37

Fig 38

Fig 39

Fig 41

Region used for the mean signal value computation

Fig 40

Frontal tube

Lateral detector

PMMA
Al-1100

Frontal detector

X-ray beams

Lateral tube

Fig 43

Fig 42

Deviation index for different PMMA/Al values

Scout 1E
Equivalent PMMA thickness only
[min, max] = [-3.38, 0.25]

Scout 2E
Known PMMA and Al thicknesses
[min, max] = [-0.26, 0.16]

Legend

○ |DI| ≤ 0.5 (target range)
□ 0.5 < |DI| ≤ 1
△ |DI| > 1 (under-/over-exposure)

EP 4 525 724 B1

Fig 44

Fig 45

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021094004 A1
- EP 16711889 A
- US 1607660 A
- EP 17758269 A
- US 16628410 B
- WO 2021094806 A
- WO 2021094404 A